# EUROPEAN PATENT SPECIFICATION

(11) **EP 1 173 594 B1**
(45) Date of publication and mention of the grant of the patent: **29.10.2008**
(21) Application number: 00922647.3
(22) Date of filing: 17.04.2000
(51) Int. Cl.: C12N 15/82, C12N 15/11, A01H 1/04

(54) **METHODS FOR DELIVERING INHIBITORY RNA TO PLANTS AND APPLICATIONS THEREOF**
VERFAHREN ZUR EINFÜHRUNG VON INHIBITORISCHER RNA IN PFLANZEN UND DEREN ANWENDUNGEN
METHODES ET MOYENS D'ADMINISTRATION D'ARN INHIBITEUR A DES VEGETAUX ET APPLICATIONS ASSOCIEES

(30) Priority: 20.04.1999 US 294022
(43) Date of publication of application: 23.01.2002
(73) Proprietor: Bayer BioScience N.V., 9052 Gent (BE)
(72) Inventor: MEULEWAETER, Frank, B-9160 Eksaarde (BE); CORNELISSEN, Marc, B-9070 Heusden (BE); JACOBS, John, B-9820 Merelbeke (BE); VAN ELDIK, Gerben, B-9000 Gent (BE); METZLAFF, Michael, B-3080 Tervuren (BE)
(86) International application number: PCT/EP2000/003521
(87) International publication number: WO 2000/063397

(56) References cited:
- WO-A-95/34668
- WO-A-98/36083
- WO-A-98/53083
- WO-A-99/36516
- HAMILTON ANDREW J ET AL: "A transgene with repeated DNA causes high frequency, post-transcriptional suppression of ACC-oxidase gene expression in tomato" PLANT JOURNAL,BLACKWELL SCIENTIFIC PUBLICATIONS, OXFORD,GB, vol. 15, no. 6, 1998, pages 737-746, XP002146504 ISSN: 0960-7412 cited in the application

### SEQUENCE LISTING

<110> Aventis CropScience N.V.
<120> Methods and means for delivering inhibitory RNA to plants and applications thereof
<130> FKOSAT
<140>
   <141>
<150> US SN 09/294022
   <151> 1999-04-20
<160> 15
<170> PatentIn Ver. 2.1
<210> 1
   <211> 3684
   <212> DNA
   <213> Artificial Sequence
<220>
   <223> Description of Artificial Sequence:cDNA copy of the nucleotide sequence of the genome of TNV-A
<400> 1
   agtattcata ccaagaatac caaataggtg caaggcctta ctcagctaaa gagtctaaaa 60
   tggagctacc aaaccaacac aagcaaacgg ccgccgaggg tttcgtatct ttcctaaact 120
   ggctatgcaa cccatggaga cgacagcgaa cagtcaacgc tgcagttgcg ttccaaaaag 180
   atcttctcgc cattgaggat tccgagcatt tggatgacat caatgagtgt ttcgaggagt 240
   ctgctggggc acaatctcag cgaactaagg ttgtcgccga cggagcatat gcccccgcaa 300
   aatccaacag gacccgccga gttcgtaagc agaagaagca caagtttgta aaatatcttg 360
   tcaacgaagc tcgtgccgag tttggattgc ccaaaccaac tgaggcaaac agacttatgg 420
   tccaacattt cttgctcaga gtgtgcaagg attggggcgt tgttactgcc cacgtacacg 480
   gcaatgttgc actagctttg ccactggtgt tcatcccaac ggaagatgat ctgctatcac 540
   gagcattgat gaacacacat gctactagag ccgctgtacg aggcatggac aatgtccaag 600
   gggaggggtg gtggaacaat aggttgggga ttgggggcca ggtcggactg gccttccggt 660
   ccaaataggg gtgccttgaa aggaggccag gattctccac gtccgtttcg cgtggggaac 720
   atcctgatct ggtggtcata ccatcagggc gccctgagaa acagcgtcag ttgttacgct 780
   atagtggtat aggcggccat ttattaatcg gcatccacaa caactctctt tccaacctgc 840
   gtaggggctt gatggaaaga gtattctatg tcgaggggcc caatgggctt caagacgccc 900
   ctaagcccgt caagggagct tttcgaaccc ttgataagtt tcgtgatctc tatactaaaa 960
   atagttggcg tcatacccct gtaactagtg aacaattcct aatgaattac acgggcagga 1020
   aactgactat ttacagagag gcggttgata gtttgtcgca tcaacccctt agctcacgag 1080
   atgcgaaact aaagacattc gtgaaggccg aaaaattaaa tctttctaag aagcctgacc 1140
   ctgctcccag ggtcatccaa cctagatcgc ctcggtataa cgtttgtttg ggcaggtacc 1200
   tccgacatta tgagcatcac gcgtttaaaa ccattgccaa gtgctttggg gaaatcacgg 1260
   tcttcaaagg gtttactctg gagcaacaag gggaaatcat gcgctcgaag tggaataaat 1320
   atgttaatcc cgtcgcagtc ggactcgacg ccagtcgttt cgaccaacac gtgtctgttg 1380
   aagcactcga gtatgagcat gaattttacc tcagagacta cccaaatgat aaacagctaa 1440
   aatggctgct aaagcagcaa ttgtgcaacg taggaacggc attcgccagt gacggcatta 1500
   taaaatacaa gaagaagggt tgtagaatga gcggagacat gaacacgagt ttgggcaact 1560
   gcattctaat gtgcgccatg gtctacgggt tgaaagaaca cttaaacatc aatttgtccc 1620
   ttgcaaataa tggggatgac tgcgtcattg tctgtgagaa agcggattta aagaaattga 1680
   caagcagcat cgagccatat ttcaagcagt ttggattcaa gatggaagtg gaaaaacccg 1740
   tggatatatt tgagcgcata gaattttgcc aaacccaacc tgtgttcgat ggatcccagt 1800
   acatcatggt acgcaaacct tctgtggtaa catctaaaga cgtcactagc cttatcccat 1860
   gtcaaacgaa agcacaatac gcagaatggc tgcaagctgt aggtgagtgt ggcatgagca 1920
   ttaacggtgg gattcctgtc atgcagaatt tctaccaaaa gctccaaact ggcatccgcc 1980
   gcacaaaatt caccaagacc ggcgagttcc agacgaacgg attggggtat cactctagat 2040
   atatgcatag agtggcccgg gttccttcgc ctgaaacccg tttatccttc tatctagctt 2100
   tcggtatcac accagacctc caagaagcat tggagatctt ctatgatacc cacaggcttg 2160
   agttggatga tgttatccca actgatacct accaagtgtc aggagagcat ttgatcaatg 2220
   gattaccaaa ctgatgtaac ggaggacaat gtgcaaatac gcggtcgggc taggagcgtt 2280
   gagggtaaga aacacaatgg ttcgggatta actggcgtta agcgtcacgc ggtgagcgaa 2340
   acatctcaga aatcacagca aggtactggc aatggaacta tgaccaatat agccgaagaa 2400
   cagaccatta ccgtgacata caactttaac ttttaagtta tggctgcgtg tcgctgttgt 2460
   gatacttcac caggtattac actattccct tactttgcaa ttctcatcct tatattggca 2520
   atacttgttg tagggactcc caatcaacaa tatcaccatt ctccaagcac ttacgagtac 2580
   aagactcaac acatttcgat cgcaaaatag acatggcagg aaagaagaac aacaacaacg 2640
   gtcagtatat aatactgcgt actccagagc aacaggtgga gatagaccag cgcaacgccc 2700
   gtcgtgctca aatgggtcgc atgaagaagg ctagacagcc cgttcagcga tacttacagc 2760
   aacacgggtt gcgaaacgga ttgtccggta gagggggcta catagtggct cccacctccg 2820
   ggggggttgt cactcgaccc atagtgccga aattctccaa caggggagat tccactatag 2880
   tccgtaacac tgagattttg aacaaccaaa tcttagcggc gctaggcgca ttcaatacaa 2940
   caaactccgc actgattgca gcagcaccat catggctggc tagcatcgct gatctttaca 3000
   gtaaatacag atggctctca tgtgagatca tctacattcc aaaatgcccc accaccacca 3060
   gtggatcaat tgccatggct ttcacatacg acagaaatga cgctgcaccc accgcaaggg 3120
   ctcagctgtc acaatcttac aaggccatca attttccacc gtatgcggga tacgacggag 3180
   cagcatattt gaattcgaac cagggagctg ggtcagccat cgccgttcaa cttgatgtta 3240
   ccaagttgga caagccatgg taccccacta tctcctctgc cggcttcggg gcgctcagcg 3300
   tcctcgatca gaaccaattc tgccccgcgt cccttgtggt cgctagcgat gggggacccg 3360
   ctactgctac tccagcaggg gaccttttca tcaagtacgt gattgagttc attgaaccaa 3420
   tcaacccaac aatgaacgtc tagttctttg tactgtaact tggctaatgc ctaaggtgga 3480
   gtcacaccat tggagacgga gacggatcct gggaaacagg cttgacgggc ggggggtggt 3540
   gcccccgacg acgcatcact ccggatacca atggtacacc actatggcag ggtctgccaa 3600
   ggtcttgtgc accaagaacc cctggaaacg ggggggaggg gggtagcaca tatcatccag 3660
   attgaggggc ctttgcccca cccc                                        3684
<210> 2
   <211> 6395
   <212> DNA
   <213> Artificial Sequence
<220>
   <223> Description of Artificial Sequence: cDNA copy of the nucleotide sequence of the genome of TMV-U1
<400> 2
   gtatttttac aacaattacc aacaacaaca aacaacaaac aacattacaa ttactattta 60
   caattacaat ggcatacaca cagacagcta ccacatcagc tttgctggac actgtccgag 120
   gaaacaactc cttggtcaat gatctagcaa agcgtcgtct ttacgacaca gcggttgaag 180
   agtttaacgc tcgtgaccgc aggcccaagg tgaacttttc aaaagtaata agcgaggagc 240
   agacgcttat tgctacccgg gcgtatccag aattccaaat tacattttat aacacgcaaa 300
   atgccgtgca ttcgcttgca ggtggattgc gatctttaga actggaatat ctgatgatgc 360
   aaattcccta cggatcattg acttatgaca taggcgggaa ttttgcatcg catctgttca 420
   agggacgagc atatgtacac tgctgcatgc ccaacctgga cgttcgagac atcatgcggc 480
   acgaaggcca gaaagacagt attgaactat acctttctag gctagagaga ggggggaaaa 540
   cagtccccaa cttccaaaag gaagcatttg acagatacgc agaaattcct gaagacgctg 600
   tctgtcacaa tactttccag acaatgcgac atcagccgat gcagcaatca ggcagagtgt 660
   atgccattgc gctacacagc atatatgaca taccagccga tgagttcggg gcggcactct 720
   tgaggaaaaa tgtccatacg tgctatgccg ctttccactt ctctgagaac ctgcttcttg 780
   aagattcata cgtcaatttg gacgaaatca acgcgtgttt ttcgcgcgat ggagacaagt 840
   tgaccttttc ttttgcatca gagagtactc ttaattattg tcatagttat tctaatattc 900
   ttaagtatgt gtgcaaaact tacttcccgg cctctaatag agaggtttac atgaaggagt 960
   ttttagtcac cagagttaat acctggtttt gtaagttttc tagaatagat acttttcttt 1020
   tgtacaaagg tgtggcccat aaaagtgtag atagtgagca gttttatact gcaatggaag 1080
   acgcatggca ttacaaaaag actcttgcaa tgtgcaacag cgagagaatc ctccttgagg 1140
   attcatcatc agtcaattac tggtttccca aaatgaggga tatggtcatc gtaccattat 1200
   tcgacatttc tttggagact agtaagagga cgcgcaagga agtcttagtg tccaaggatt 1260
   tcgtgtttac agtgcttaac cacattcgaa cataccaggc gaaagctctt acatacgcaa 1320
   atgttttgtc ctttgtcgaa tcgattcgat cgagggtaat cattaacggt gtgacagcga 1380
   ggtccgaatg ggatgtggac aaatctttgt tacaatcctt gtccatgacg ttttacctgc 1440
   atactaagct tgccgttcta aaggatgact tactgattag caagtttagt ctcggttcga 1500
   aaacggtgtg ccagcatgtg tgggatgaga tttcgctggc gtttgggaac gcatttccct 1560
   ccgtgaaaga gaggctcttg aacaggaaac ttatcagagt ggcaggcgac gcattagaga 1620
   tcagggtgcc tgatctatat gtgaccttcc acgacagatt agtgactgag tacaaggcct 1680
   ctgtggacat gcctgcgctt gacattagga agaagatgga agaaacggaa gtgatgtaca 1740
   atgcactttc agagttatcg gtgttaaggg agtctgacaa attcgatgtt gatgtttttt 1800
   cccagatgtg ccaatctttg gaagttgacc caatgacggc agcgaaggtt atagtcgcgg 1860
   tcatgagcaa tgagagcggt ctgactctca catttgaacg acctactgag gcgaatgttg 1920
   cgctagcttt acaggatcaa gagaaggctt cagaaggtgc tttggtagtt acctcaagag 1980
   aagttgaaga accgtccatg aacggttcga tggccagagg agagttacaa ttagctggtc 2040
   ttgctggaga tcatccggag tcgtcctatt ctaagaacga ggagatagag tctttagagc 2100
   agtttcatat ggcaacggca gattcgttaa ttcgtaagca gatgagctcg attgtgtaca 2160
   cgggtccgat taaagttcag caaatgaaaa actttatcga tagcctggta gcatcactat 2220
   ctgctgcggt gtcgaatctc gtcaagatcc tcaaagatac agctgctatt gaccttgaaa 2280
   cccgtcaaaa gtttggagtc ttggatgttg catctaggaa gtggttaatc aaaccaacgg 2340
   ccaagagtca tgcatggggt gttgttgaaa cccacgcgag gaagtatcat gtggcgcttt 2400
   tggaatatga tgagcagggt gtggtgacat gcgatgattg gagaagagta gctgtcagct 2460
   ctgagtctgt tgtttattcc gacatggcga aactcagaac tctgcgcaga ctgcttcgaa 2520
   acggagaacc gcatgtcagt agcgcaaagg ttgttcttgt ggacggagtt ccgggctgtg 2580
   ggaaaaccaa agaaattctt tccagggtta attttgatga agatctaatt ttagtacctg 2640
   ggaagcaagc cgcggaaatg atcagaagac gtgcgaattc ctcagggatt attgtggcca 2700
   cgaaggacaa cgttaaaacc gttgattctt tcatgatgaa ttttgggaaa agcacacgct 2760
   gtcagttcaa gaggttattc attgatgaag ggttgatgtt gcatactggt tgtgttaatt 2820
   ttcttgtggc gatgtcattg tgcgaaattg catatgttta cggagacaca cagcagattc 2880
   catacatcaa tagagtttca ggattcccgt accccgccca ttttgccaaa ttggaagttg 2940
   acgaggtgga gacacgcaga actactctcc gttgtccagc cgatgtcaca cattatctga 3000
   acaggagata tgagggcttt gtcatgagca cttcttcggt taaaaagtct gtttcgcagg 3060
   agatggtcgg cggagccgcc gtgatcaatc cgatctcaaa acccttgcat ggcaagatcc 3120
   tgacttttac ccaatcggat aaagaagctc tgctttcaag agggtattca gatgttcaca 3180
   ctgtgcatga agtgcaaggc gagacatact ctgatgtttc actagttagg ttaaccccta 3240
   caccagtctc catcattgca ggagacagcc cacatgtttt ggtcgcattg tcaaggcaca 3300
   cctgttcgct caagtactac actgttgtta tggatccttt agttagtatc attagagatc 3360
   tagagaaact tagctcgtac ttgttagata tgtataaggt cgatgcagga acacaatagc 3420
   aattacagat tgactcggtg ttcaaaggtt ccaatctttt tgttgcagcg ccaaagactg 3480
   gtgatatttc tgatatgcag ttttactatg ataagtgtct cccaggcaac agcaccatga 3540
   tgaataattt tgatgctgtt accatgaggt tgactgacat ttcattgaat gtcaaagatt 3600
   gcatattgga tatgtctaag tctgttgctg cgcctaagga tcaaatcaaa ccactaatac 3660
   ctatggtacg aacggcggca gaaatgccac gccagactgg actattggaa aatttagtgg 3720
   cgatgattaa aaggaacttt aacgcacccg agttgtctgg catcattgat attgaaaata 3780
   ctgcatcttt agttgtagat aagttttttg atagttattt gcttaaagaa aaaagaaaac 3840
   caaataaaaa tgtttctttg ttcagtagag agtctctcaa tagatggtta gaaaagcagg 3900
   aacaggtaac aataggccag ctcgcagatt ttgattttgt agatttgcca gcagttgatc 3960
   agtacagaca catgattaaa gcacaaccca agcaaaaatt ggacacttca atccaaacgg 4020
   agtacccggc tttgcagacg attgtgtacc attcaaaaaa gatcaatgca atatttggcc 4080
   cgttgtttag tgagcttact aggcaattac tggacagtgt tgattcgagc agatttttgt 4140
   ttttcacaag aaagacacca gcgcagattg aggatttctt cggagatctc gacagtcatg 4200
   tgccgatgga tgtcttggag ctggatatat caaaatacga caaatctcag aatgaattcc 4260
   actgtgcagt agaatacgag atctggcgaa gattgggttt tgaagacttc ttgggagaag 4320
   tttggaaaca agggcataga aagaccaccc tcaaggatta taccgcaggt ataaaaactt 4380
   gcatctggta tcaaagaaag agcggggacg tcacgacgtt cattggaaac actgtgatca 4440
   ttgctgcatg tttggcctcg atgcttccga tggagaaaat aatcaaagga gccttttgcg 4500
   gtgacgatag tctgctgtac tttccaaagg gttgtgagtt tccggatgtg caacactccg 4560
   cgaatcttat gtggaatttt gaagcaaaac tgtttaaaaa acagtatgga tacttttgcg 4620
   gaagatatgt aatacatcac gacagaggat gcattgtgta ttacgatccc ctaaagttga 4680
   tctcgaaact tggtgctaaa cacatcaagg attgggaaca cttggaggag ttcagaaggt 4740
   ctctttgtga tgttgctgtt tcgttgaaca attgtgcgta ttacacacag ttggacgacg 4800
   ctgtatggga ggttcataag accgcccctc caggttcgtt tgtttataaa agtctggtga 4860
   agtatttgtc tgataaagtt ctttttagaa gtttgtttat agatggctct agttgttaaa 4920
   ggaaaagtga atatcaatga gtttatcgac ctgacaaaaa tggagaagat cttaccgtcg 4980
   atgtttaccc ctgtaaagag tgttatgtgt tccaaagttg ataaaataat ggttcatgag 5040
   aatgagtcat tgtcagaggt gaaccttctt aaaggagtta agcttattga tagtggatac 5100
   gtctgtttag ccggtttggt cgtcacgggc gagtggaact tgcctgacaa ttgcagagga 5160
   ggtgtgagcg tgtgtctggt ggacaaaagg atggaaagag ccgacgaggc cactctcgga 5220
   tcttactaca cagcagctgc aaagaaaaga tttcagttca aggtcgttcc caattatgct 5280
   ataaccaccc aggacgcgat gaaaaacgtc tggcaagttt tagttaatat tagaaatgtg 5340
   aagatgtcag cgggtttctg tccgctttct ctggagtttg tgtcggtgtg tattgtttat 5400
   agaaataata taaaattagg tttgagagag aagattacaa acgtgagaga cggagggccc 5460
   atggaactta cagaagaagt cgttgatgag ttcatggaag atgtccctat gtcgatcagg 5520
   cttgcaaagt ttcgatctcg aaccggaaaa aagagtgatg tccgcaaagg gaaaaatagt 5580
   agtaatgatc ggtcagtgcc gaacaagaac tatagaaatg ttaaggattt tggaggaatg 5640
   agttttaaaa agaataattt aatcgatgat gattcggagg ctactgtcgc cgaatcggat 5700
   tcgttttaaa tatgtcttac agtatcacta ctccatctca gttcgtgttc ttgtcatcag 5760
   cgtgggccga cccaatagag ttaattaatt tatgtactaa tgccttagga aatcagtttc 5820
   aaacacaaca agctcgaact gtcgttcaaa gacaattcag tgaggtgtgg aaaccttcac 5880
   cacaagtaac tgttaggttc cctgacagtg actttaaggt gtacaggtac aatgcggtat 5940
   tagacccgct agtcacagca ctgttaggtg cattcgacac tagaaataga ataatagaag 6000
   ttgaaaatca ggcgaacccc acgactgccg aaacgttaga tgctactcgt agagtagacg 6060
   acgcaacggt ggccataagg agcgcgataa ataatttaat agtagaattg atcagaggaa 6120
   ccggatctta taatcggagc tctttcgaga gctcttctgg tttggtttgg acctctggtc 6180
   ctgcaacttg aggtagtcaa gatgcataat aaataacgga ttgtgtccgt aatcacacgt 6240
   ggtgcgtacg ataacgcata gtgtttttcc ctccacttaa atcgaagggt tgtgtcttgg 6300
   atcgcgcggg tcaaatgtat atggttcata tacatccgca ggcacgtaat aaagcgaggg 6360
   gttcgaatcc ccccgttacc cccggtaggg gccca                            6395
<210> 3
   <211> 1245
   <212> DNA
   <213> Artificial Sequence
<220>
   <223> Description of Artificial Sequence: cDNA copy of the nucleotide sequence of the gnome of STNV-2
<400> 3
   agtaaagaca ggaaacttta ccgactatca gaatgacaaa acgtcaaagc aaacaatcaa 60
   accgcaagag cgttgcatca caggtgcgta gtattgttga gtcaatggct gagcagaagc 120
   gatttgcttt tcttacgaac accaacacag tcactacagc aggtaccgtg atcaacctga 180
   gcaacaacat cgtgcaagga gatgaccttg ttaatcgcac cggagaccag attaagacca 240
   tacaccagac tttattgact cggtgtacag gaattaccaa cagccaaagc tttcggttca 300
   tctggtttcg tgacaacacc aataggggga ctacaccggc tgtgactgag gtgttagaca 360
   gtgctagtat aacatcccag tataacccca ctacgttcca gcaaaagagg ttcactgttt 420
   tccaagattt catgttggat acctctatag ttggacgtgt gattgtccat cggactgccg 480
   ttgataagaa acggcgtgcg atattttaca acggtgctgc ttctgtagcc gcgtcaaatg 540
   gccccggtgc cacatttgta cttgtcattg gatcacatgc cactggacag tatgatgtga 600
   cagccgagat tgtttatctg gacatgtaga ccatggtcat gatgatgata gtgaaggacg 660
   ctgaaagatg cgtagctacc ctcctggtgc acttcctggt gcaaagcaga accaaagggt 720
   acggtggtac ggcggacagt agtcctgaac tagtaaatca ggaccgggag aaaaccagct 780
   gacggctaaa tccattccca ctagtgtatt agtggaacga ggccccgcgt gaattggggt 840
   ggctgcatgg ggtggaaaac catgtggtcg cagtcatttc tcctatgcat tattgtctca 900
   atacttgtgt gcaacaatgc tgttaatcaa cgtagcactc aacatcactt caaaaccccc 960
   tccatgtcac aagaatcaag atgcatgtct gtgtttagcg gtatatattt tgcatccact 1020
   tgatcgtgat tttgccctgg gcacctcgcg cggttggtac ccgcggagac tccccacagc 1080
   aacatggcat taggcaggga taaggtatag tgactagaca aatgcgcgtg aagctggaaa 1140
   gtccggttag cagtggggtt gtgcggaatg cagcctcaac aaggtatagc tgctgcatag 1200
   gagatgtgaa cctttcaaac ttgaattcaa gtctcatgac tgccc 1245
<210> 4
   <211> 1058
   <212> DNA
   <213> Artificial Sequence
<220>
   <223> Description of Artificial Sequence: cDNA copy of the nucleotide sequence of the genome of STMV
<400> 4
   agtaaactta ccaatcaaaa gacctaacca acaggactgt cgtggtcatt tatgctgttg 60
   ggggacatag ggggaaaaca tattgccttc ttctacaaga ggccttcagt cgccataatt 120
   acttggcgcc caattttggg tttcagttgc tgtttccagc tatggggaga ggtaaggtta 180
   aaccaaaccg taaatcgacg ggtgacaatt cgaatgttgt tactatgatt agagctggaa 240
   gctatcctaa ggtcaatccg actccaacgt gggtcagagc catacctttc gaagtgtcag 300
   ttcaatctgg tattgctttt aaagtaccgg tcgggtcact attttcggca aatttccgga 360
   cagattcctt tacaagcgtc acagtgatga gtgtccgtgc ttggacccag ttaacaccgc 420
   cagtaaatga gtacagtttt gtgaggctga agccattgtt caagactggt gactctactg 480
   aggagttcga agggcgtgca tcaaacatca acacacgagc ttctgtaggg tacaggattc 540
   caactaattt gcgtcagaat actgtggcag ccgacaatgt atgcgaagta agaagcaact 600
   gtcgacaagt cgccttggtt atttcgtgtt gttttaactg aacctcgaca taagcctttt 660
   ggatcgaagg ttaaacgatc cgctcctcgc ttgagcttga ggcggcgtat ctcttatgtc 720
   aacagagaca ctttggtcta tggttgtata acaatagata gactcccgtt tgcaagatta 780
   gggttaacag atcttgccgt tagtctggtt agcgcgtaac cggccttgat ttatggaata 840
   gatccattgt ccaatggctt tgccaatgga acgccgacgt ggctgtataa tacgtcgttg 900
   acaagtacga aatcttgtta gtgtttttcc ctccacttaa atcgaagggt tttgttttgg 960
   tcttcccgaa cgcatacgtt agtgtgacta ccgttgttcg aaacaagtaa aacaggaagg 1020
   gggttcgaat ccctccctaa ccgcgggtaa gcggccca 1058
<210> 5
   <211> 6355
   <212> DNA
   <213> Artificial Sequence
<220>
   <223> Description of Artificial Sequence: cDNA copy of the nucleotide sequence of the genome of TMV-U2
<400> 5
   gatgttttaa tagttttcga caacaacaat taaaacaaaa acaacatatt acaaacaaca 60
   aacaacaaca atggcacaca tacaatctat aattagcaac gcccttcttg aaagcgtgag 120
   tggtaaaaac actctcgtta atgaccttgc aagaaggcgc atgtacgata cggccgtgga 180
   agaatttaac gcccgcgacc gtagaccaaa ggtcaacttt tccaaaacta ttagcgaaga 240
   gcaaacgctt ctagtctcca acgcgtaccc ggagttccag attacctttt ataatactca 300
   aaatgccgta cacagtttgg ctggaggttt gagagcatta gaattggaat atctgatgct 360
   acaagttccc tatggatcgc cgacatatga tataggtggg aactttgcag cacatttgtt 420
   caaaggcagg gattacgtgc attgctgtat gcccaatctg gacatacgag atataatgag 480
   gcacgaagga caaaaggact caattgagat gtatttgtcc agattgtctc gttctaacaa 540
   ggtaattcct gagtttcaaa gggaggcttt taacaggtat gcagaagctc ccaacgaagt 600
   ctgctgctct aaaacttttc aggattgtcg aatacatccg ccagagaata gtggtagaag 660
   atacgctgtt gctctgcaca gtttgtatga tattcctgtg catgagtttg gagctgcgtt 720
   aatatctaag aatatacatg tatgttatgc agcttccatt ttggcagaag cattattact 780
   agaccagacg gaggttacgc ttaatgaaat aggcgcaact ttcaaaagag aaggtgatga 840
   tgtttctttt ttctttgctg atgaaagtac tttaaattat agtcataaat acaaaaatat 900
   cttgcattat gtagttaaat cttactttcc tgcttctagt agaatagttt actttaagga 960
   atttttagtc actagggtta atacttggtt ttgtaaattt accaaagtag atacctatat 1020
   tctgtacaag agtgttagac aagtagggtg tgatagtgat cagttctatg aggcgatgga 1080
   agacgccttt gcttacaaga aaaccttggc catgttcaac actgaaagag caatctttag 1140
   agacacggct tcggttaact tttggttccc taagatgaag gacatggtga tagtaccgct 1200
   gtttgagggt tctattacca gcaaaaagat gacaaggagt gaggtcattg ttaatcgtga 1260
   cttcgtttac acagtgctta atcatatcag aacatatcaa gccaaagcgt taacttacca 1320
   gaacgtatta tctttcgtgg agtctataag atcccgcgtg ataatcaatg gtgttactgc 1380
   taggtctgaa tgggatgtag ataaagcaat tcttcaaccc ttgtcaatga ctttcttctt 1440
   gcagactaag ctggctgcgc ttcaagacga tatagtaatg ggaaagtttc ggtgcttgga 1500
   taagaccact tctgaactta tttgggatga ggtgggcaaa ttttttggaa acgttttccc 1560
   cactatcaaa gagagattgg tgagcaggaa aattctggat gtaagtgaga atgctctgaa 1620
   gatcaagatc ccagatctgt atgtcacatg gaaagacagg ttcgtagctg aatacaccaa 1680
   gtctgaggag ttaccgcatc tagatatcaa gaaggactta gaagaagctg agcaaatgta 1740
   cgacgcgtta tcagaattat ctatccttaa gggtgctgat aatttcgata tcgcgaagtt 1800
   caaagacatg tgcaaggctt tagatgttag tcctgatgtg gcagcacgag taatcgttgc 1860
   agtggccgag aatagaagcg gtttaactct tacttttgat aagccaaccg aggagaatgt 1920
   ggctaaggct cttaaaagca cggcgtctga ggccgtggta tgtcttgaac cgacatccga 1980
   agaggtgaac gtaaataaat tttctattgc tgagaaaggg agattgcctg tgtgtgcaga 2040
   aagtcatggt ttgacgaatg ctaacttaga gcaccaggag ttggagtccc tcaacgattt 2100
   ccataaggct tgcgtggata gtgtgattac aaagcaaatg gcatcggttg tctacactgg 2160
   ctcactcaaa gttcaacaaa tgaagaacta tgtggacagt ttggcagctt cgttgtccgc 2220
   cactgtatca aatctatgca agtcactaaa ggatgaagtc gggtatgatt ctgattccag 2280
   ggagaaagtt ggtgtttggg atgtcacttt gaaaaagtgg ctcctcaaac ctgcggccaa 2340
   aggtcattca tggggagttg tcctggatta caaggggaaa atgtttactg cacttctatc 2400
   ttatgaagga gatagaatgg tgactgagag cgactggagg agggtggctg tatcatctga 2460
   tacaatggta tattctgata ttgcaaagct ccaaaatctg aggaaaacaa tgagagacgg 2520
   tgaaccccac gaacctactg caaagatggt acttgtggat ggggtgcctg gttgtggaaa 2580
   gtacaaagga gattttgaaa gatttgatct tgatgaggat ttgatcttgg ttcctggaaa 2640
   acaagctgct gctatgatca gaagaagggc taattcatct ggactgataa gagccacaat 2700
   ggacaatgtg agaacggtag attcacttct aatgcatcca aaaccgcgat cacacaagag 2760
   gctttttatt gatgaagggt tgatgctgca caccggttgt gttaacttcc tggtgcttat 2820
   ctctggttgc gacatcgcat acatttacgg agatacacag cagattcctt tcattaacag 2880
   agttcagaat ttcccgtatc ccaaacattt tgagaagctg caagtggatg aagttgagat 2940
   gaggaggacc acactgagat gcccaggtga tgtgaatttt ttcctacaat cgaagtacga 3000
   aggagcggtg acaaccactt caactgtaca acgatcggtc tcatctgaga tgataggcgg 3060
   taagggagta ctaaacagtg tttccaaacc actaaaaggg aaaattgtaa ctttcactca 3120
   ggctgataaa tttgagttag aggagaaggg ctataagaat gtgaacaccg ttcatgagat 3180
   ccaaggagaa acctttgaag atgtgtcgct ggtcagattg acggcaactc cactgactct 3240
   gatttccaag tcttccccgc atgttctagt cgctctgact agacacacaa agagcttcaa 3300
   atattacacc gtagtgttag atcctttagt acagataatt agtgatttgt cttctttaag 3360
   ctccttcctt ttagaaatgt atatggtaga agcaggtagt agatagcaat tacagatgga 3420
   tgcagtgttc aaaggtcata atctctttgt ggcaacacct aaatcaggag actttccaga 3480
   tctacagttc tattacgatg tatgcctccc tggtaatagt actatactta acaagtatga 3540
   tgctgttacc atgaggttac gtgataatag tcttaatgtg aaggattgtg ttcttgattt 3600
   ttccaaaagt attccgatgc caaaggaggt gaaaccatgt ctagagccag ttttgcgtac 3660
   cgcggcggaa ccgccaaggg ctgcaggact actcgaaaat ctggttgcaa tgattaaaag 3720
   aaatttcaac gcaccagacc tgacggggac gattgacatt gagagcaccg catctgttgt 3780
   agtagataag ttttttgata gctattttat taaaaaagaa aaatacacaa aaaatattgc 3840
   tggagtgatg acgaaggatt caatgatgag atggttggaa aacaggaaag aagtactatt 3900
   ggacgacttg gctaactaca attttacaga tctgccggcc atcgatcagt acaagcacat 3960
   gatcaaggct caaccaaaac agaaattgga cctttcaatt cagaatgaat accctgctct 4020
   gcaaacaatt gtctaccatt cgaagcagat caacggtatt ttggccggtt tctcagagct 4080
   tacaaggttg ctgctcgagg catttgattc taagaagttt cttttcttta ctaggaaaac 4140
   tccagaacag attcaagaat ttttctcgga tctcgactcg cacgttccta tggatgtgtt 4200
   agaactggat atttctaagt atgataagtc acagaacgag tttcattgtg ctgtagagta 4260
   tgaaatatgg aaaagattgg gtctcaatga gtttttggcc gaagtgtgga aacaagggca 4320
   caggaaaaca actttgaagg attacattgc tggaatcaag acatgtctgt ggtatcaaag 4380
   gaaaagcggt gatgtgacta ctttcatcgg caatactgtt ataatagcag cttgcttggg 4440
   ttcaatgtta ccgatggaaa aggtcataaa aggtgctttt tgtggagacg attccgtttt 4500
   gtattttcca aagggtttgg atttccctga cattcagtca tgtgctaatc tcatgtggaa 4560
   ttttgaggcc aaactgtata gaaagaggta cggttacttt tgtggtagat acatcataca 4620
   ccatgataag ggagcaatag tgtattatga tcctttgaag ttgatctcca aacttggggc 4680
   aaaacatatc aaggattatg atcacttaga agagttaagg gtgtctttgt gcgatgttgc 4740
   ttgttcgctc ggaaactggt gcttaggctt tccgcagctg aacgcagcta tcaaggaggt 4800
   tcataaaacc gcgattgatg gttcgtttgc ttttaattgt gttaacaaat ttttgtgtga 4860
   taaattttta tttagaactt tgtttttaaa tggctgttag tctcagagat actgtcaaaa 4920
   ttagcgagtt cattgatctt tcgaaacagg atgagatact tccggcattc atgactaagg 4980
   tcaagagtgt tagaatatcg actgtggaca agattatggc tgttaagaat gatagtcttt 5040
   ctgatgtaga tttacttaaa ggtgttaagt tagttaagaa agggtatgtg tgcttagctg 5100
   atttggtagt gtctggggag tggaatctcc cggataactg ccgtggtggt gtcagtgttt 5160
   gtattgtaga taagagaatg aaaaggagta aggaagcaac gctgggtgcg tatcacgccc 5220
   ctgcttgcaa aaagaatttt tcttttaagc taatccctaa ttattcaata acatccgagg 5280
   atgctgagaa gcacccgtgg caagtgttag tgaatatcaa aggagtggct atggaagaag 5340
   gatactgtcc tttatctttg gagttcgttt caatttgtgt agtacataaa aataatgtaa 5400
   gaaaaggttt gagggaacgt attttgagtg tgacagacgg ctcgccaatt gaactcactg 5460
   aaaaggttgt tgaggagttc gtggatgaag taccaatggc tgtgaaactc gaaaaggttc 5520
   cggaaaacaa aaaagaaatg gtaggtaata atgttaataa taagaaaata aataacagtg 5580
   gtaagaaggg ttttaaaatt gaggaaattg aggataatgt aagtgatgac gagtctatcg 5640
   cgtcatcgag tacgttttaa tcaatatgcc ttatacaatc aactctccga gccaatttgt 5700
   ttacttatct tccgcttacg cagatcctgt gcagctgatc aatctgtgta caaatgcatt 5760
   gggtaaccag tttcaaacgc aacaagctag gacaacagtc caacagcaat ttgcggatgc 5820
   ctggaaacct gtgcctagta tgacagtgag atttcctgca tcggatttct atgtgtatag 5880
   atataattcg acgcttgatc cgttgatcac ggcgttatta aatagctttg atactagaaa 5940
   tagaataata gaggttgata atcaacccgc accgaatact actgaaatcg ttaacgcgac 6000
   tcagagggta gacgatgcta ctgtagctat aagggcttca atcaataatt tggctaatga 6060
   actggttcgt ggaactggca tgttcaatca agcaggcttt gagactgcta gtggacttgt 6120
   ctggaccaca actccggcta cttagctatt gttgtgagat ttcctaaaat aaagtcgctg 6180
   aagacttaaa attcagggtg gctgatacca aaatcagcag tggttgttcg tccacttaaa 6240
   tataacgatt gtcatatctg gatccaacag ttaaaccatg tgatggtgta tactgtggta 6300
   tggcgtaaaa catcggagag gttcgaatcc tcccctaacc gccggtagcg gccca 6355
<210> 6
   <211> 2346
   <212> DNA
   <213> Artificial Sequence
<220>
   <223> Description of Artificial Sequenc : nucleotide sequence of the tomato phytoene desaturase (pds) encoding cDNA
<400> 6
   cttttactag ttatagcatt cggtatcttt ttctgggtaa ctgccaaacc accacaaatt 60
   acaagtttcc atttaactct tcaacttcaa cccaaccaaa tttatttcct taattgtgca 120
   gaaccactcc ctatatcttc taggtgcttt cattcgttcc gaggtaagaa aagatttttg 180
   tttctttgaa tgctttatgc cactcgttta acttctgagg tttgtggatc ttttaggcga 240
   cttttttttt ttttgtatgt aaaatttgtt tcataaatgc ttctcaacat aaatcttgac 300
   aaagagaagg aattttacca agtatttagg ttcagaaatg gataattttc ttactgtgaa 360
   atatccttat ggcaggtttt actgttattt ttcagtaaaa tgcctcaaat tggacttgtt 420
   tctgctgtta acttgagagt ccaaggtagt tcagcttatc tttggagctc gaggtcgtct 480
   tctttgggaa ctgaaagtcg agatggttgc ttgcaaagga attcgttatg ttttgctggt 540
   agcgaatcaa tgggtcataa gttaaagatt cgtactcccc atgccacgac cagaagattg 600
   gttaaggact tggggccttt aaaggtcgta tgcattgatt atccaagacc agagctggac 660
   aatacagtta actatttgga ggctgcattt ttatcatcaa cgttccgtgc ttctccgcgc 720
   ccaactaaac cattggagat tgttattgct ggtgcaggtt tgggtggttt gtctacagca 780
   aaatatttgg cagatgctgg tcacaaaccg atactgctgg aggcaaggga tgttctaggt 840
   ggaaaggtag ctgcatggaa agatgatgat ggagattggt acgagactgg tttgcatata 900
   ttctttgggg cttacccaaa tattcagaac ctgtttggag aattagggat taacgatcga 960
   ttgcaatgga aggaacattc aatgatattt gcaatgccaa gcaagccagg agaattcagc 1020
   cgctttgatt tctccgaagc tttacccgct cctttaaatg gaattttagc catcttaaag 1080
   aataacgaaa tgcttacatg gccagagaaa gtcaaatttg caattggact cttgccagca 1140
   atgcttggag ggcaatctta tgttgaagct caagatggga taagtgttaa ggactggatg 1200
   agaaagcaag gtgtgccgga cagggtgaca gatgaggtgt tcattgctat gtcaaaggca 1260
   ctcaacttta taaaccctga cgaactttca atgcagtgca ttttgatcgc attgaacagg 1320
   tttcttcagg agaaacatgg ttcaaaaatg gcctttttag atggtaatcc tcctgagaga 1380
   ctttgcatgc cgattgttga acacattgag tcaaaaggtg gccaagtcag actgaactca 1440
   cgaataaaaa agattgagct gaatgaggat ggaagtgtca agagttttat actgagtgac 1500
   ggtagtgcaa tcgagggaga tgcttttgtg tttgccgctc cagtggatat tttcaagctt 1560
   ctattgcctg aagactggaa agagattcca tatttccaaa agttggagaa gttagtcgga 1620
   gtacctgtga taaatgtaca tatatggttt gacagaaaac tgaagaacac atatgatcat 1680
   ttgctcttca gcagaagctc actgctcagt gtgtatgctg acatgtctgt tacatgtaag 1740
   gaatattaca accccaatca gtctatgttg gaattggttt ttgcacctgc agaagagtgg 1800
   atatctcgca gcgactcaga aattattgat gcaacgatga aggaactagc aacgcttttt 1860
   cctgatgaaa tttcagcaga tcaaagcaaa gcaaaaatat tgaagtacca tgttgtcaaa 1920
   actccgaggt ctgtttataa aactgtgcca ggttgtgaac cctgtcggcc tttacaaaga 1980
   tccccaatag aggggtttta tttagccggt gactacacga aacagaaata cttggcttca 2040
   atggaaggcg ctgtcttatc aggaaagctt tgtgctcaag ctattgtaca ggattatgag 2100
   ttacttgttg gacgtagcca aaagaagttg tcggaagcaa gcgtagttta gctttgtggt 2160
   tattatttag cttctgtaca ctaaatttat gatgcaagaa gcgttgtaca caacatatag 2220
   aagaagagtg cgaggtgaag caagtaggag aaatgttagg aaagctccta tacaaaagga 2280
   tggcatgttg aagattagca tctttttaat cccaagttta aatataaagc atattttatg 2340
   gaattc                                                            2346
<210> 7
   <211> 7096
   <212> DNA
   <213> Artificial Sequence
<220>
   <223> Description of Artificial Sequence: nucleotide sequence of the tobacco nitrate reductase (nia-2) encoding cDNA
<400> 7
   tacatacaag ggcgcgaata aacttttttt aaagtaaatg tatatgaact tgcaatgaaa 60
   gaggacctta acttgtttgt ctttgttgct ttctgcaaat ttcaccttaa cagcccattt 120
   gagattgatt tagttagtta taacaattag ttaaatgctt gtgtaatttg aagaaaatat 180
   ttggacgtgc tcgctgaaaa cattatactc ctatataata gaaatacttt ctgaaaagtt 240
   ggtcttgttc aaaaacgtat aagagagttg gtcttctcat aaatagtcac tagctttctg 300
   attttttttc actttctata tcacgtaaat aggtactcaa atttgatatt tacaccaaac 360
   aaatgaaaat aggatatgtg tttttcatac gtatatttat ctatcgtact taatgataca 420
   tacatataca tataacctta ctttttgatt actaaaaatt taattatatt taatttgggt 480
   aaatatcaga tgccacaaaa catttaccta gccactgttt ttgactacta aaaatttaat 540
   tatgtttagc ttgggtaaat atcagatgtc actaaacatt ttacctagcc attcctccga 600
   aaagaaattg agaaggaaat tagagttagt ggagccataa taatgtttaa tgtgaccata 660
   actcggtgaa aaccacggca agaataagaa acagctgtta aggctaacca acagctgcat 720
   atctttaagc catttgctat taccccaaca tcgcatcttc ctctgatccc gaccctacgg 780
   gcgtaaaaag tgtaaatcgt tagaattgtt ttatttattt tatgatgtca ctatttttta 840
   aaatcaaaat taaattgggg tgtcgatttt tttgggtcct gcttatgtat agtatggcgc 900
   tatggaggca ctgagagagt ccgaaacgtt tctatataag gccaccccac gcattcacaa 960
   acttcgttcc caaacagaac aagaaaatca aatctcggag agagagagag agaaatattt 1020
   tgagagagaa atacagaaaa tctctcttcc ttctttcctt tttttttcaa tccccattca 1080
   tattcttttt ttagaataat ctatggcggc atctgtcgaa aacaggcagt tcagtcacct 1140
   agaagccggt ttatcccggt ctttcaagcc ccggtctgat tccccggttc gtggctgcaa 1200
   cttcccttcg cccaacagta ctaatttcca aaagaaacca aattccacca tttaccttga 1260
   ttactcgtcg agtgaagacg acgatgatga tgacgaaaaa aatgagtacc ttcaaatgat 1320
   taaaaaaggg aattcagagt tagagccatc tgttcatgac actagggacg aaggtaccgc 1380
   tgataattgg attgaacgca acttttccat gattcgtctc accggaaagc atccatttaa 1440
   ctccgaacca ccgttgaacc ggctcatgca ccacggcttt atcacaccgg tcccacttca 1500
   ttacgttcgt aaccatggac cggttcccaa gggcacgtgg gatgactgga ccgtggaagt 1560
   cacgggacta gtgaagcgtc ctatgaaatt cacaatggac cagttggtta acgaattccc 1620
   ttgtagagaa ttgcccgtta cgcttgtttg tgctggcaat cgaaggaaag aacagaacat 1680
   ggttaaacaa accattggtt tcaactgggg cgccgctgcc gtttcaacaa cgatatggcg 1740
   cggggtaccc ctccgcgctt tgctaaaacg gtgcggtgtt tttagcaaga ataaaggggc 1800
   gcttaatgtt tgcttcgaag gagctgatgt gttgcccgga ggtggtggtt caaagtatgg 1860
   aaccagcatt aagaaggaat ttgcaatgga tccagcacga gatatcatcg tagcctacat 1920
   gcagaacgga gaaaaattgg cacccgacca cgggtttcca gtacgaatga taattccagg 1980
   attcattgga ggaagaatgg tgaaatggat aaagaggatt atagtcacca cccaagaatc 2040
   agacagctat tatcatttca aggacaatag agttcttcct ccccatgttg atgctgaact 2100
   tgcaaatacc gaaggtacgt accgtaacta tttcaattta ttactccatt tgttccaatt 2160
   tatgtgaacc tatttccttt ttggtccgtt caaaaaagaa tgaacccttt ctaaatttgg 2220
   taacaattta gcttaaactt acaacttcac ccttaatgag aaacttttat aaccacacaa 2280
   ataccctggg gcccatttgg acttgtttag gtcgacaaat tccaaaagtt ttattttttt 2340
   cttaaacttc gtgctcagtc aaacaggttc acgtaaattg aaacggagag agtatcattt 2400
   ttattaaggg gtataaatat attttaatta gttgagactt gcacatacaa gtaaaatatt 2460
   tcttagaata caaaatcaac tgaaagctta cttctaatta tatggttttg aattttcctt 2520
   tcaatgaagt aaataaaaag gaaacaatta tattcaacgc atgtaggtat atggtcctgt 2580
   cattatctca aatcaaatgg tttaaagaca aaggactttg gaaacataga attgtcagct 2640
   ttatagttat ggagtactat attagttagc tgtttgcatc tattcataat tggtctatct 2700
   gtgtgcagca tggtggtaca agccagagta tatcatcaat gagcttaata ttaactctgt 2760
   cattacgacg ccgtgtcatg aagaaatttt gccaattaac gcctggacga ctcagcgacc 2820
   ttacacgttg aggggctatt cttattctgg ttagtatttt tatattttcc gattttgctg 2880
   agaatatcat atttcttagt tttgtcgata catcgtatcc tctaactctg acgttttact 2940
   tcgtccttat gcacccactt acgtccttac tttctcagac agtttattga tgaaaactac 3000
   ttactatttt cgacccgata gcctcagcgt ccttaattaa atgtgatgtt ttgaaagaga 3060
   tattctctcc cgtctatttt aattaatttt tggctgtttt tatacgtggg aatctatttt 3120
   taacattaat taatatagaa atgaaccata ttaatattat taatttcttc attgaaaata 3180
   caacaaatac tcttcggctc ttactacaat gacaatttgg aagaaaaata attaattcct 3240
   tcctaatatc tgaaaaatca aatattgtgg accataaaaa aaggtcaaaa aattaattaa 3300
   aatgaactgg agagagtaaa ttagaaaata taattatagc actagtaatt aaagttatta 3360
   gatgtcttct ttaaaaagcg tgtgaaaact ttaaagacga aatataatat gaatattatc 3420
   taatacttag aaagtgtcaa taattggtag acaatttaaa ctatatacta gttaaaaagt 3480
   ctgtcaatac aactattagt attggggatt agagagaata gtagtaaaat ggagtaattg 3540
   gacgcatgag cttgggcatg ctgattgctg tcagcttgtt tgctaatgtg aaaaagaaaa 3600
   tagtaagaaa aggccaacat ggttttgttt attttattat gtggtagtac acaaaaacct 3660
   ggggagcttt cctagttctg aagagtcggt ctttggtagc acaaaattaa tagtatagta 3720
   taccaagtga atattaaatt caattgtcta aagcacggaa tctttttgac tactttagtt 3780
   cctgcatctt gggttgcctc aacaacaccc tttattgaat tattatagta atgttcaata 3840
   taatatacaa ttagaaaaca ctctaagtgg tcactttata tggatctagt caatactatt 3900
   tcttctaaac aacgtgccta attacttccc actttccagt acatgaccac cattaagttt 3960
   aatttttgtc aattccttgt gcaattggcc cttcaaatga gcagaagtgt tacgtaggaa 4020
   aactaacttc agctactatt ataggagtaa acctgttagg aaaagatgct cgaggaactg 4080
   acaaaacttg tagaataatt agccattgta ttgattgaaa tactgattgt gaacgtgtaa 4140
   caaacaggcg gagggaaaaa agtaacgcga gtagaagtga cgttggatgg aggagaaaca 4200
   tggcaagtta gcacactaga tcacccagag aagcccacca aatatggcaa gtactggtgt 4260
   tggtgctttt ggtcactcga ggttgaggtg ttagacttgc tcagtgctaa agaaattgct 4320
   gttcgagctt gggatgagac cctcaatact caacccgaga agcttatttg gaacgtcatg 4380
   gtacgttcac ttcttctttt acctttattt cttttaactt ctatatacta gcggtgtaaa 4440
   gttattttac accataagtt aacttacaaa aatatgtaac tatttatact acgagtgatg 4500
   agggcaagaa ggggtttaag tatttgacaa taaatgtaaa ccctgcaatt ttgttcctaa 4560
   ttttttatcc tttcaactct ttgtgattgc ttcattatct agattcacag agcacatgtg 4620
   ttcacatgcc aaaacaaaaa actacaaaca aaaaaacttt tcactagctt tagtctaaga 4680
   ttcccctttt tttttttggg aggtgtgtgg tccatactcc atagatcaat tccagccact 4740
   gacgtaccaa accctgaaaa ttcctagtag ttatagcgac gtacaatcat ttcatattat 4800
   gtaagcagag acgtgatcac atgaactaga tgtgaatacc acttgcccag tccaccaggt 4860
   caattcatct agatgtgtaa atcttgacac cagcactggg tcacttttat aacactagca 4920
   tttaacaaca tttcatcctt gaacattact tgggctaatt aataagtatt tttttttata 4980
   tactctaaaa attgtaatta cataaatgaa tttaacttat acacgctgac aatgttacta 5040
   attccacttt ttacggacgg ttatctatag aaatcattta ggtgaaacaa ttctcttaca 5100
   ctatgatcag tgttagtaca taatggttat tacattttct aaatattgtg ctatgttgca 5160
   atgttcaggg aatgatgaat aattgctggt tccgagtaaa gatgaatgtg tgcaagcctc 5220
   acaagggaga gattggaata gtgtttgagc atccgactca acctggaaac caatcaggtg 5280
   gatggatggc gaaggagaga catttggaga tatcagcaga ggcacctcaa acactaaaga 5340
   agagtatctc aactccattc atgaacacag cttccaagat gtactccatg tccgaggtca 5400
   ggaaacacag ctctgctgac tctgcttgga tcatagtcca tggtcatatc tatgacgcca 5460
   cgcgtttctt gaaagatcac cctggtggga ctgacagcat tctcatcaat gctggcactg 5520
   attgcactga ggaatttgat gcaattcatt ctgataaggc taagaagctc ttggaggatt 5580
   tcaggattgg tgaactcata actactggtt acacctctga ctctcctggc aactccgtgc 5640
   acggatcttc ttccttcagc agctttctag cacctattaa ggaacttgtt ccagcgcaga 5700
   ggagtgtggc cctaattcca agagagaaaa tcccatgcaa actcatcgac aagcaatcca 5760
   tctcccatga tgttaggaaa tttcgatttg cattgccctc tgaggatcaa gtcttgggct 5820
   tgcctgttgg aaaacatatc ttcctctgtg ccgttattga cgataagctc tgcatgcgcg 5880
   cttacacgcc tactagcacg atcgatgagg tggggtactt cgagttggtt gtcaagatat 5940
   acttcaaagg aattcaccct aaattcccca atggagggca aatgtcacag tatcttgatt 6000
   ctatgccgtt agggtcattt ctcgacgtga aaggtccatt aggtcacatt gaataccaag 6060
   gaaagggaaa tttcttagtt catggcaaac agaagtttgc caagaagttg gccatgatag 6120
   caggtggaac aggaataact ccagtgtatc aagtcatgca ggcaattctg aaagatccag 6180
   aagatgacac agaaatgtat gtggtgtatg ctaacagaac agaggatgat attttactta 6240
   aggaagagct tgattcatgg gctgagaaaa ttccagagag ggttaaagtt tggtatgtgg 6300
   ttcaggattc tattaaagaa ggatggaagt acagcattgg ttttattaca gaagccattt 6360
   tgagagaaca tatccctgag ccatctcaca caacactggc tttggcttgt ggaccacctc 6420
   ctatgattca atttgctgtt aatccaaact tggagaagat gggctatgac attaaggatt 6480
   ccttattggt gttctaattt taaaaacaaa acaatatctg caggaataaa tttttttttt 6540
   ccccctatca gttgtacata ttgtatttgg tttatcaccc ccatgtacta cgtagtgttt 6600
   gtagttctta catttttatt ttttagaatt tttttaaacc ttaggatata aaggttttct 6660
   cttccaacaa agtgattctt tagggaagaa atgtactgta ctgtactagt atgtctaagc 6720
   cgaaagttgt aatgtttacc atgacaaatt gtattcaatt cctcatggaa tagtaacatt 6780
   gtgttcatgt gtcttcctgt aagcgatctt caaaatatca atgtatatat atagtaattg 6840
   caaaccattg ttccttttcc cgatgtagtt aactactctt tctttagctt ctagtctctg 6900
   gtgaatattt ttttttctat aactctttaa ttaatacggc cttaaataag agaaaagttt 6960
   aaaccacgaa tatcattatg cagacgtata ggtaattaat ctactttttg aaaaaaaatc 7020
   tattttcttt atgtggtcct tcaaaataat attctagaac cttttgtata ttccctttta 7080
   acttctattt agtttt                                                  7096
<210> 8
   <211> 1839
   <212> DNA
   <213> Artificial Sequence
<220>
   <223> Description of Artificial Sequence: nucleotide sequence of the tobacco nitrite reductase (nir-1) encoding cDNA
<400> 8
   tttctattaa atttctggca ccttcattgc caaatccagc tagattttcc aagaatgctg 60
   tcaagctcca cgcaactccg ccgtctgtgg cagcgccgcc agctggtgct ccagaggttg 120
   ctgctgagag gctagaaccc agagttgagg aaaaagatgg ttattggata ctcaaggagc 180
   agtttagaaa aggcataaat cctcaagaaa aggtcaagat tgagaagcaa cctatgaagt 240
   tgttcatgga aaatggtatt gaagagcttg ctaagatacc cattgaagag atagatcagt 300
   ccaagcttac taaggatgat attgatgtta ggcttaagtg gcttggcctc ttccatagga 360
   gaaagaacca atatgggcgg ttcatgatga gattgaagct tccaaatgga gtaacaacga 420
   gtgcacagac tcgatacttg gcgagtgtga taaggaaata cgggaaagaa ggatgtgctg 480
   atattacaac gaggcaaaat tggcagattc gtggagttgt actgcctgat gtgcccgaga 540
   tactaaaggg actagcagaa gttgggttga ccagtttgca gagtggcatg gacaatgtca 600
   ggaatccagt aggaaatcct cttgctggaa ttgatccaga agaaatagta gacacagggc 660
   cttacactaa tttgctctcc caatttatca ctggcaattc acgaggcaat cccgcagttt 720
   ctaacttgcc aaggaagtgg aatccgtgcg tagtaggctc tcatgatctt tatgaacatc 780
   cccatatcaa cgatctcgcg tacatgcctg ccacgaaaga tggacgattt ggattcaacc 840
   tgcttgtggg tgggttcttc agcgcaaaaa gatgtgatga ggcaattcct cttgatgcat 900
   gggttccagc tgatgatgtt gttccggttt gcaaagcaat actggaagct tttagagatc 960
   ttggtttcag agggaacaga cagaaatgta gaatgatgtg gttaatcgat gaactgggtg 1020
   tagaaggatt cagggcagag gtcgagaaga gaatgccaca gcaagagcta gagagagcat 1080
   ctccagagga cttggttcag aaacaatggg aaagaagaga ttatcttggt gtacatccac 1140
   aaaaacaaga aggctacagc tttattggtc ttcacattcc agtgggtcgt gttcaagcag 1200
   acgatatgga tgagctagct cgtttagctg atgagtatgg ttcaggagag atccggctta 1260
   ctgtggaaca aaacattatt attcccaaca ttgagaactc aaagattgag gcactgctca 1320
   aagagcctgt tctgagcaca ttttcacctg atccacctat tctcatgaaa ggtttagtgg 1380
   cttgtactgg taaccagttt tgtggacaag ccataatcga gactaaagct cgttccctga 1440
   tgataactga agaggttcaa cggcaagttt ctttgacacg gccagtgagg atgcactgga 1500
   caggctgccc gaatacgtgt gcacaagttc aagttgcgga cattggattc atgggatgcc 1560
   tgactagaga taagaatgga aagactgtgg aaggcgccga tgttttctta ggaggcagaa 1620
   tagggagtga ttcacatttg ggagaagtat ataagaaggc tgttccttgt gatgatttgg 1680
   taccacttgt tgtggactta ctagttaaca actttggtgc agttccacga gaaagagaag 1740
   aaacagaaga ctaataaaat ttagaatagt tggtgatttt gctgtgttca taacatgtaa 1800
   tgtatgataa atcaatgcaa acatttctac ctacgtgag                        1839
<210> 9
   <211> 1294
   <212> DNA
   <213> Artificial Sequence
<220>
   <223> Description of Artificial Sequence: DNA of the beta-1,3-glucanase of Nicotiana plumbagenifolia
<400> 9
   ttgctcttca aatggctgct attatactgc taggattgct tgtttccagc actgagatag 60
   taggagctca atcagtaggt gtttgctacg gaatgctggg caacaacttg ccaccagcat 120
   cacaagttgt acaactgtac aagtcaaaaa acataagaag aatgaggctt tatgatccaa 180
   atcaagcagc tttacaggct ttaagaggct ccaacattga agttatgtta ggagttccca 240
   attcagatct ccaaaacatt gctgctaacc cctcaaatgc aaataattgg gtccagagga 300
   atgtcagaaa tttctggcca gccgttaaat ttaggtacat tgccgttgga aatgaagtca 360
   gccctgtaac aggcacatct tcacttaccc gatatcttct tccggccatg aggaacattc 420
   ggaatgcgat ttcttcagca ggtttgcaaa acaatatcaa agtctcaagt tctgtagaca 480
   tgaccttgat tgggaactct tttccaccat cacagggttc gtttaggaac gacgttaggt 540
   cgttcattga tccgattatt gggtttgtaa ggcgcataaa ttcgccttta ctcgttaaca 600
   tttatcctta ttttagctat gctggtaatc cgcgcgatat ttctctcccc tatgctcttt 660
   tcactgctcc aaatgtggtg gtacaagatg gttcacttgg atatagaaac ttatttgatg 720
   caatgtcgga tgctgtgtat gctgccctgt ctcgagccgg agggggctcg atagagattg 780
   ttgtgtccga gagtggctgg ccatctgctg gcgcatttgc cgcgacaaca aacaatgcag 840
   caacttacta caagaactta attcagcatg ttaaaagggg tagtccaaga aggcctaata 900
   aagtcattga gacctattta tttgctatgt ttgatgagaa taacaaaaac cctgaattgg 960
   agaaacattt tggactcttt tcccccaaca agcagcccaa atatccactc agctttgggt 1020
   tttcagatag atattgggac atttctgctg aaaataatgc tactgcagct tctctcataa 1080
   gtgagatgtg ataagagagt tctctttaaa tatctttaca tggatggaaa acttagtacc 1140
   aataactaga ttgtttcttt ctttatgcaa ttttcttgta atgagagact agtacttgct 1200
   ctctgtgtcc ttgtggagag taactagaga caaattaagc aaataacata aataattgag 1260
   tgttgattct gcaatgataa atagaaaaaa aaaa                             1294
<210> 10
   <211> 720
   <212> DNA
   <213> Artificial Sequence
<220>
   <223> Description of Artificial Sequence: green fluorescent protein encoding regon
<400> 10
   atggtgagca agggcgagga gctgttcacc ggggtggtgc ccatcctggt cgagctggac 60
   ggcgacgtaa acggccacaa gttcagcgtg tccggcgagg gcgagggcga tgccacctac 120
   ggcaagctga ccctgaagtt catctgcacc accggcaagc tgcccgtgcc ctggcccacc 180
   ctcgtgacca ccctgaccta cggcgtgcag tgcttcagcc gctaccccga ccacatgaag 240
   cagcacgact tcttcaagtc cgccatgccc gaaggctacg tccaggagcg caccatcttc 300
   ttcaaggacg acggcaacta caagacccgc gccgaggtga agttcgaggg cgacaccctg 360
   gtgaaccgca tcgagctgaa gggcatcgac ttcaaggagg acggcaacat cctggggcac 420
   aagctggagt acaactacaa cagccacaac gtctatatca tggccgacaa gcagaagaac 480
   ggcatcaagg tgaacttcaa gatccgccac aacatcgagg acggcagcgt gcagctcgcc 540
   gaccactacc agcagaacac ccccatcggc gacggccccg tgctgctgcc cgacaaccac 600
   tacctgagca cccagtccgc cctgagcaaa gaccccaacg agaagcgcga tcacatggtc 660
   ctgctggagt tcgtgaccgc cgccgggatc actctcggca tggacgagct gtacaagtaa 720
<210> 11
   <211> 1809
<212> DNA
   <213> Artificial Sequence
<220>
   <223> Description of Artificial Sequence:beta-glucuronidase encoding region.
<400> 11
   atggtccgtc ctgtagaaac cccaacccgt gaaatcaaaa aactcgacgg cctgtgggca 60
   ttcagtctgg atcgcgaaaa ctgtggaatt gatcagcgtt ggtgggaaag cgcgttacaa 120
   gaaagccggg caattgctgt gccaggcagt tttaacgatc agttcgccga tgcagatatt 180
   cgtaattatg cgggcaacgt ctggtatcag cgcgaagtct ttataccgaa aggttgggca 240
   ggccagcgta tcgtgctgcg tttcgatgcg gtcactcatt acggcaaagt gtgggtcaat 300
   aatcaggaag tgatggagca tcagggcggc tatacgccat ttgaagccga tgtcacgccg 360
   tatgttattg ccgggaaaag tgtacgtatc accgtttgtg tgaacaacga actgaactgg 420
   cagactatcc cgccgggaat ggtgattacc gacgaaaacg gcaagaaaaa gcagtcttac 480
   ttccatgatt tctttaacta tgccggaatc catcgcagcg taatgctcta caccacgccg 540
   aacacctggg tggacgatat caccgtggtg acgcatgtcg cgcaagactg taaccacgcg 600
   tctgttgact ggcaggtggt ggccaatggt gatgtcagcg ttgaactgcg tgatgcggat 660
   caacaggtgg ttgcaactgg acaaggcact agcgggactt tgcaagtggt gaatccgcac 720
   ctctggcaac cgggtgaagg ttatctctat gaactgtgcg tcacagccaa aagccagaca 780
   gagtgtgata tctacccgct tcgcgtcggc atccggtcag tggcagtgaa gggcgaacag 840
   ttcctgatta accacaaacc gttctacttt actggctttg gtcgtcatga agatgcggac 900
   ttacgtggca aaggattcga taacgtgctg atggtgcacg accacgcatt aatggactgg 960
   attggggcca actcctaccg tacctcgcat tacccttacg ctgaagagat gctcgactgg 1020
   gcagatgaac atggcatcgt ggtgattgat gaaactgctg ctgtcggctt taacctctct 1080
   ttaggcattg gtttcgaagc gggcaacaag ccgaaagaac tgtacagcga agaggcagtc 1140
   aacggggaaa ctcagcaagc gcacttacag gcgattaaag agctgatagc gcgtgacaaa 1200
   aaccacccaa gcgtggtgat gtggagtatt gccaacgaac cggatacccg tccgcaagtg 1260
   cacgggaata tttcgccact ggcggaagca acgcgtaaac tcgacccgac gcgtccgatc 1320
   acctgcgtca atgtaatgtt ctgcgacgct cacaccgata ccatcagcga tctctttgat 1380
   gtgctgtgcc tgaaccgtta ttacggatgg tatgtccaaa gcggcgattt ggaaacggca 1440
   gagaaggtac tggaaaaaga acttctggcc tggcaggaga aactgcatca gccgattatc 1500
   atcaccgaat acggcgtgga tacgttagcc gggctgcact caatgtacac cgacatgtgg 1560
   agtgaagagt atcagtgtgc atggctggat atgtatcacc gcgtctttga tcgcgtcagc 1620
   gccgtcgtcg gtgaacaggt atggaatttc gccgattttg cgacctcgca aggcatattg 1680
   cgcgttggcg gtaacaagaa agggatcttc actcgcgacc gcaaaccgaa gtcggcggct 1740
   tttctgctgc aaaaacgctg gactggcatg aacttcggtg aaaaaccgca gcagggaggc 1800
   aaacaatga                                                         1809
<210> 12
   <211> 411
   <212> DNA
   <213> Artificial Sequence
<220>
   <223> Description of Artificial Sequence: cDMA copy of part of the region of a TMV-U2 variant comprising the origin of assembly
<400> 12
   ccctcgccaa ttgaactcac tgaaaaagtt gttgatgagt tcgtagatga agtaccgatg 60
   gctgtgaaac tcgaaaggtt ccggaaaaca aaaaagagag tggtaggtaa taatgttaat 120
   aataagaaaa taaataatag tggtaagaag ggtttgaaag ttgaggaaat tgaggataat 180
   gtaagtgatg acgagtctat cgcgtcatcg agtacgtttt aatcaatatg ccttatacaa 240
   tcaactctcc gagccaattt gtttacttaa gttccgctta tgcagatcct gtgcagctga 300
   tcaatctgtg tacaaatgca ttaggtaacc agtttcaaac gcaacaagct aggacaacag 360
   tccaacagca atttgcggat gcctggaaac ctgtgcctag tatgacagtg a          411
<210> 13
   <211> 198
   <212> DNA
   <213> Artificial Sequence
<220>
   <223> Description of Artificial Sequence: cDNA copy of STMV leader region
<400> 13
   agtaaaactt accaatcaaa agacctaacc aacaggactg tcgtggtcat ttatgctgtt 60
   gggggacata gggggaaaac atattgcctt cttctacaag aggccttcag tcgccataat 120
   tacttggcgc ccaattttgg gtttcagttg ctgtttccag ctatggggag aggtaaggtt 180
   aaaccaaacc gtaaatcg                                               198
<210> 14
   <211> 455
   <212> DNA
   <213> Artificial Sequence
<220>
   <223> Description of Artificial Sequence:cDNA copy of STMV trailer region
<400> 14
   gacaagtcgc cttggttatt tcgtgttgtt ttaactgaac ctcgacataa gccttttgga 60
   tcgaaggtta aacgatccgc tcctcgcttg agcttgaggc ggcgtatctc ttatgtcaac 120
   agagacactt tggtctatgg ttgtataaca atagatagac tcccgtttgc aagattaggg 180
   ttaacagatc ttgccgttag tctggttagc gcgtaaccgg ccttgattta tggaatagat 240
   ccattgtcca atggctttgc caatggaacg ccgacgtggc tgtataatac gtcgttgaca 300
   agtacgaaat cttgttagtg tttttccctc cacttaaatc gaagggtttt gttttggtct 360
   tcccgaacgc atacgttagt gtgactaccg ttgttcgaaa caagtaaaac aggaaggggg 420
   ttcgaatccc tccctaaccg cgggtaagcg gccca                            455
<210> 15
   <211> 1971
   <212> DNA
   <213> Artificial Sequence
<220>
   <223> Description of Artificial Sequence: cDNA copy of part of the genome of a TMV-U1 variant, comprising MP and CP genes
<400> 15
   ggaaacactg tgattatagc tgcatgtttg gcctcgatgc ttccgatgga gaaaataatc 60
   aaaggagcct tttgtggtga cgatagtctg ctgtacttcc caaagggttg tgagtttccg 120
   gatgtgcaac actccgcgaa tcttatgtgg aattttgaag caaaactgtt taaaaaacag 180
   tatggatact tttgcggaag gtatgtaata catcacgaca gaggatgcat tgtgtattac 240
   gatcccctaa agttgatctc gaaacttggt gctaaacaca tcaaggattg ggaacacttg 300
   gaggagttca gaaggtctct ttgtgatgtt gctgtttcgt tgaacaattg tgcgtattac 360
   acacagttgg acgacgctgt atgggaggtt cataagaccg cccctccagg ttcgtttgtt 420
   tataaaagtc tggtgaagta tttgtctgat aaagttcttt ttagaagttt gtttatagat 480
   ggctctagtt gttaaaggaa aagtgaatat caatgagttt atcgacctga caaaaatgga 540
   gaagatctta ccgtcgatgt ttacccctgt aaagagtgtc atgtgttcca aagttgataa 600
   aataatggtt catgagaatg agtcattgtc agaggtaaac cttctcaaag gagttaagct 660
   tattgatagt ggatacgtct gtttagccgg tttggtcgtc acgggcgagt ggaacttgcc 720
   tgacaattgc agaggaggtg tgagcgtgtg tctggtggac aaaaggatgg aaagagccga 780
   cgaggccact ctcggatctt actacacagc agctgcaaag aaaagatttc agttcaaggt 840
   cgttcccaat tatgctataa ccacccagga cgcgatgaaa aacgtctggc aagttttagt 900
   caatattaga aatgtaaaga tgtcagcggg tttctgtccg ctttctctgg agtttgtgtc 960
   ggtgtgtatc gtttatagaa ataatataaa attaggtttg agagagaaga tcacaagtgt 1020
   gagagatgga gggcccatgg aacttacaga agaagttgtt gatgagttca tggaagatgt 1080
   ccctatgtca atcaggcttg caaagtttcg atctcgaacc ggaaaaaaga gtgatgtccg 1140
   taaagggaaa attagtagta gtgatcggtc agcgccgaac aagaactata gaaatgttaa 1200
   ggattttgga ggaatgagtt ttaaaaagaa taatttaatc gatgatgatt cggagactac 1260
   tgtcgccgaa tcggattcgt tttaaatatg tcttacagta tcactactcc atctcagttc 1320
   gtgttcttgt cagcagcgtg ggccgaccca atagagttaa ttaatttatg tactaatgcc 1380
   ttaggaaatc agtttcaaac acaacaagct cgaactgtcg ttcaaagaca attcagtgag 1440
   gtgtggaaac cttcaccaca agtgactgtt aggttccctg acagtgactt taaggtgtac 1500
   aggtacaatg cggtattaga cccgctagtc acagcactgt taggtgcatt tgacactaga 1560
   aatagaataa tagaagttga aaatcaggcg aaccccacaa ctgccgaaac gttagatgct 1620
   actcgtagag tagacgacgc aacggtggcc ataaggagcg ctataaataa tttagtagta 1680
   gaattgatca gaggaaccgg atcttataat cggagctctt tcgagagctc ttctggtttg 1740
   gtttggaact ctggtcctgc aacttgaggt agtcaagatg cataataaat aacggattgt 1800
   gtccgtaatc acacgtggtg cgtacgataa cgcatagtgt ttttccctcc acttaaatcg 1860
   aagggttgtg tcttggatcg cgcgggtcaa atgtatatgg ttcatataca tccgcaggca 1920
   cgtaataaag cgaggggttc gaatcccccc gttacccccg gtaggggccc a           1971

## Description

### Field of the invention.

This invention relates to the field of functional genomics in plants, more particularly it relates to methods for the further identification and isolation of a nucleic acid with a nucleotide sequence of interest in a collection of preselected nucleic acid sequences correlated with a particular trait, preferably an agronomical important trait, using a kit of viral RNA vectors, wherein one of the viral RNA vectors comprises a library of gene-silencing constructs for the preselected nucleic acid sequences. The invention also relates to a method for modulating, preferably reducing, particularly eliminating the expression of a selected nucleic acid sequence, using the viral vector kit, whereby one of the vectors comprises a gene-silencing construct for the selected nucleic acid sequence. The latter method may be used for validating the function of a nucleic acid sequence whose expression is correlated with the presence or absence of a specific trait in plants, but with otherwise unknown function. Preferably, one of the viral RNA vector components of the kit is a vector derived from a satellite virus.

### Background art

The recent, rapid expansion of available nucleic acid sequence information has necessitated the development of methods for identifying the function of nucleic acid sequences, particularly transcribed nucleic acid sequences such as expressed sequence tags, with unknown function, in an efficient and labor-cost effective way.

To identify the role of sequenced nucleic acids from plants of unknown function it is necessary to produce or identify plants in which those nucleic acids are either structurally or functionally inactivated. Plants wherein predetermined nucleic acid sequences are structurally inactivated can be generated using recombination technologies such as homologous recombination as described by Kempin et al. (1997) or using specific technologies such as the use of mixed duplex oligonucleotides (chimeraplasts) to generate specific mutations (as described in WO 96/22364 and WO 99/07865). Alternatively, plants with a mutation in a predetermined nucleotide sequence can be identified by screening a saturated mutant library, such as but not limited to a T-DNA insertion library or a transpon insertion library (see e.g. Pereira and Aerts, 1998). These methodologies all require the generation of a large number of permanently altered plants, and thus are less amenable for application in high throughput methods. Moreover, the recovery of plants with recessive mutations in essential genes requires time-consuming breeding to maintain the plants in heterozygous state. Maintenance of dominant lethal mutations in essential genes is virtually impossible.

Plants with functionally inactivated predetermined nucleotide sequences can be generated in a straightforward way using methodologies wherein inhibitory RNA is generated, such as antisense or sense RNA.

The use of inhibitory RNA to reduce or abolish gene expression, also known as gene silencing, is well established in the art and is the subject of several reviews (e.g Baulcombe 1996, Stam et al. 1997, Depicker and Van Montagu, 1997). Several patent applications relate to the practical exploitation of gene silencing.

US 5,190,131 and EP 0 467 349 A1 describe methods and means to regulate or inhibit gene expression in a cell by incorporating into or associating with the genetic material of the cell a non-native nucleic acid sequence which is transcribed to produce an mRNA which is complementary to and capable of binding to the mRNA produced by the genetic material of that cell.

EP 0 240 208 describes a method to regulate expression of genes encoded for in plant cell genomes, achieved by integration of a gene under the transcriptional control of a promoter which is functional in the host and in which the transcribed strand of DNA is complementary to the strand of DNA that is transcribed from the endogenous gene(s) one wishes to regulate.

EP 0 223 399 A1 describes methods to effect useful somatic changes in plants by causing the transcription in the plant cells of negative RNA strands which are substantially complementary to a target RNA strand. The target RNA strand can be a mRNA transcript created in gene expression, a viral RNA, or other RNA present in the plant cells. The negative RNA strand is complementary to at least a portion of the target RNA strand to inhibit its activity *in vivo*.

EP 0 647 715 A1 and US patents 5, 034,323, 5,231,020 and 5,283,184 describe methods and means for producing plants exhibiting desired phenotypic traits, by selecting transgenotes that comprise a DNA segment operably linked to a promoter, wherein transcription products of the segment are substantially homologous to corresponding transcripts of endogenous genes, particularly endogenous flavonoid biosynthetic pathway genes.

WO 93/23551 describes methods and means for the inhibition of two or more target genes, which comprise introducing into the plant a single control gene which has distinct DNA regions homologous to each of the target genes and a promoter operative in plants adapted to transcribe form such distinct regions RNA that inhibits expression of each of the target genes.

A major disadvantage of these technologies, which hampers the exploitation thereof in high throughput gene function discovery methods, is the intrinsic unpredictability and low occurrence of the gene silencing phenomenon.

Recently, Waterhouse et al. (1998) have described methods and means to make gene silencing in plants more efficient and predictable, by simultanous expression of both sense and antisense constructs in cells of one plant. The sense and antisense nucleic acids may be in the same transcriptional unit, so that a single RNA transcript that has self-complementarity is generated upon transcription.

Hamilton et al. (1998) describe improved silencing e.g. of tomato ACC-oxidase gene expression using a sense RNA containing two additional upstream inverted copies of its 5' untranslated region.

WO 98/53083 describes constructs and methods for enhancing the inhibition of a target gene within an organism, involving the insertion into the gene silencing vector of an inverted repeat of all or part of a polynucleotide region within the vector.

It should be clear however, that the use of inhibitory RNA as a tool in reversed genetics analysis of gene function via high throughput methods, whereby the inhibitory RNA is generated from gene-silencing constructs which are stably integrated in the genome of transgenic plants, suffers from the same drawbacks as the methods wherein the nucleotide sequences are structurally inactivated.

EP 0 194 809 and US 5,500,360 suggest the use of viral RNA vectors to produce regulatory RNA such as anti-sense RNA.

Initial exploration of the use of viral vectors to deliver inhibitory RNA into cells of plants has been described by Chapman (1991). In this publication, gene silencing constructs comprising nucleotide sequences complementary to the translated region of the GUS gene on a PVX derived viral vector were described. The experiments however, remained inconclusive as to whether gene silencing could be provoked using viral vectors for the production of inhibitory RNA.

WO 93/03161 is directed toward recombinant plant viral nucleic acids and to hosts infected thereby. The non-native nucleic acid sequence which is transcribed may be transcribed as an RNA which is capable of regulating the expression of a phenotypic trait by an anti-sense mechanism.

English et al., 1996 describe the suppression of the accumulation of a viral vector comprising a foreign nucleotide sequence in transgenic plants exhibiting silencing of nuclear genes comprising the same foreign nucleotide sequences, thus linking gene silencing and viral vectors, albeit in a reverse way as envisioned here.

Kumagai et al. 1995 (PNAS 92, 1679-1683) described the inhibition of phytoene desaturase gene by viral delivery of antisense RNA.

WO 95/34668 suggests the use of genetic constructs based on RNA viruses which replicate in the cytoplasm of cells to provide inhibitory RNA, either antisense or co-suppressor (sense) RNA.

Baulcombe et al. (1998) and Ruiz et al. (1998) describe virus-induced gene silencing of the endogenous phytoene desaturase gene (PDS) or of a green fluorescent protein transgene (GFP) in plants, using potato virus X derived vectors carrying inserts homologous to PDS and GFP, respectively. The authors further suggested that virus-induced gene silencing may develop into a novel assay of gene function, by introducing a fragment of the genome of a viral vector and inferring the function of the gene from the symptoms of the infected plants exhibiting gene silencing.

The described methods for identification of the function of a gene with known nucleotide sequence however, have drawbacks and limitations. In the first place, the applicability of the mentioned viral RNA vector based gene silencing methods on larger scale is in practice limited to the identification of genes with essential functions or genes with macroscopically visible phenotypes. Secondly, all methods employ viral vectors which are capable of autonomous replication in plant cells and cell-to-cell movement, whereby care has to be taken not to inactivate the essential functions required for these functions. This may particularly be a disadvantage when tailoring these methods to the needs of particular plants, such as crop plants, by developing new viral vectors more apt for replication and systemic spread in the plants.

The prior art is thus deficient in the lack of efficient methods for large scale identification of the function of nucleic acids with known nucleotide sequence, or for the isolation of the genes of interest from a pool of genes with known nucleotide sequence, but unknown function.

### Brief Description of the figures

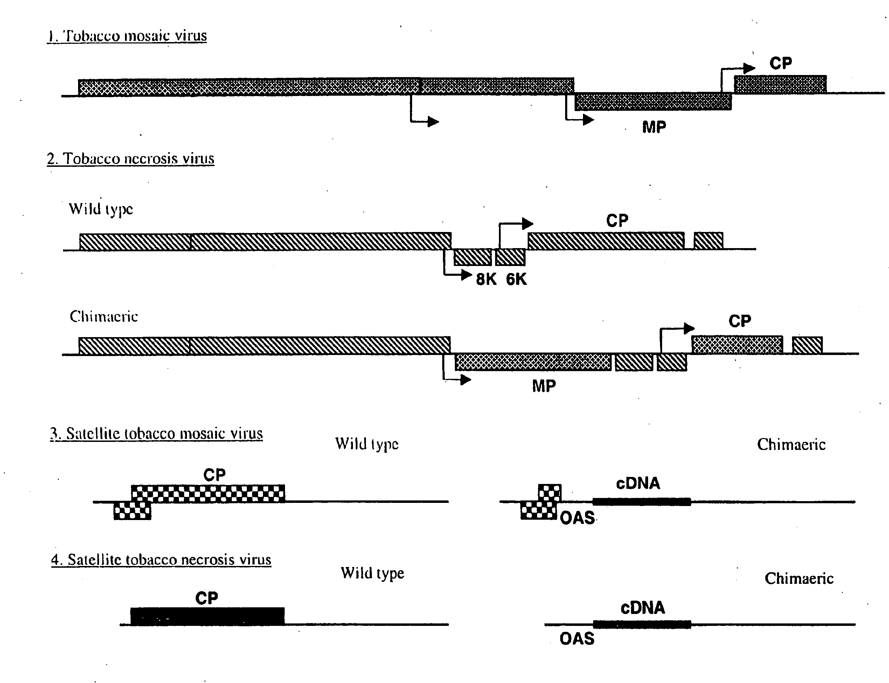

Figure 1 is a schematic representation of the viral RNA vectors used in the Examples. MP: movement protein; CP: coat protein; OAS: origin of assembly. The open reading frames are indicated by boxes. Each original viral genome is characterized by a specific pattern.
   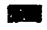 : tobacco mosaic virus; 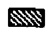 tobacco necrosis virus; 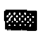 satellite tobacco mosaic virus; 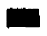 satellite tobacco necrosis virus.

### Summary of the invention

The invention provides a method for isolating genes involved in the determination of a trait or a phenotype of a plant species, comprising identifying a set of nucleic acid sequences of genes, whose expression is correlated with a trait of interest; creating a library of gene silencing constructs, being nucleic acids, which when transcribed yield RNA molecules comprising sense RNA or antisense RNA or both, comprising a nucleotide sequence of at least 100 nucleotides having at least 75% sequence identity with the nucleic acid sequences of the genes, whose expression is correlated with the trait of interest in a viral RNA vector comprising at least cis elements from a satellite RNA virus recognized by a replicase from a corresponding helper virus and capable of replicating in a plant cell only when provided externally with essential functions required for said replication; infecting a collection of individual plants of the same plant species with the library of gene silencing constructs and with a corresponding helper virus or helper virus RNA, whereby each plant is infected with at least one member of the library and with the helper virus or helper virus RNA; identifying a plant wherein the trait or phenotype is altered using an assay adapted to that trait or phenotype; and isolating the gene involved in the determination of the trait or phenotype in the plant species, from the library, based on the nucleotide sequence to which the gene silencing construct in the identified plant was targeted.

Preferably the satellite RNA virus is satellite tobacco mosaic virus or satellite tobacco necrosis virus, and the viral RNA vector further comprises an origin of assembly of tobacco mosaic virus.Preferably the helper virus is tobacco mosaic virus or tobacco necrosis virus which comprises a gene encoding a coat protein gene of tobacco mosaic virus.

Gene-silencing constructs comprised within the viral RNA vector may comprise antisense RNA or sense RNA of at least 100 nucleotides in length Particularly the gene-silencing constructs comprise a complementary stretch of at least 50, preferably at least 100, nucleotides of sense and antisense RNA. Especially preferred are gene-silencing constructs comprising at least two copies of part of the nucleotide sequences of the collection of nucleic acids, the copies being in inverted repeat.

It is another object of the invention to provide a method for the isolation or selection of a nucleic acid with a specific function from a collection of nucleic acids, wherein the collection of nucleic acids is characterized by the fact that variation in the expression pattern of the nucleic acids is correlated with variation in a trait or phenotype of a plant harboring the nucleic acids comprising the steps of creating a library of gene silencing constructs, being nucleic acids, which when transcribed yield RNA molecules comprising sense RNA or antisense RNA or both, comprising a nucleotide sequence of at least 100 nucleotides having at least 75% sequence identity with the nucleotide sequence of said nucleic acids the variation in the expression pattern of which is correlated with variation in a trait/phenotype of a plant harboring said nucleic acids in a viral RNA vector comprising at least cis elements from a satellite RNA virus recognized by a replicase from a corresponding helper virus and capable of replicating in a plant cell only when provided externally with essential functions required for said replication; infecting a collection of plants with the library of gene silencing constructs and with a corresponding helper virus or helper virus RNA, whereby each plant is infected with one member of the library and with the helper virus or helper virus RNA; identifying plants with altered trait or phenotype using an assay adapted to the trait or phenotype under investigation; and optionally isolating the nucleic acid with the specific function from the identified plant with altered trait or phenotype.

It is yet another object of the invention to provide a method for determining the function encoded by a nucleic acid comprising a known nucleotide sequence in a plant, comprising the steps of providing a viral RNA vector comprising at least cis elements from a satellite RNA virus recognized by a replicase from a corresponding helper virus and capable of replicating in a plant cell only when provided externally with essential functions required for said replication comprising a gene-silencing construct being a nucleic acid, which when transcribed yields RNA molecules comprising sense RNA or antisense RNA or both, comprising a nucleotide sequence of at least 100 nucleotides having at least 75% sequence identity with the known nucleotide sequence; infecting or inoculating the plant with the chimaeric viral RNA vector and a corresponding helper virus or helper virus RNA; and identifying an altered trait or phenotype of the co-infected plant.

The invention further provides a method for identifying essential genes from a plant, comprising creating a library of random gene-silencing constructs by cloning random DNA or cDNA fragments of at least 100 nucleotides in length, particularly duplicated cDNA fragments of at least 100 nucleotides in length in inverted repeat, in a cDNA copy of a viral RNA vector comprising at least cis elements from a satellite RNA virus, preferably STMV or STNV, particularly a viral RNA vector comprising an origin of assembly of TMV, recognized by a replicase from a corresponding helper virus and capable of replicating in a plant cell only when provided externally with essential functions required for said replication; infecting a plant with individual members of the library and with a corresponding helper virus, preferably tobacco mosaic virus or tobacco necrosis virus comprising the coat protein of tobacco mosaic virus; identifying plants developing a gene-silencing-construct-associated phenotype, particularly necrosis and optionally isolating the viral RNA vector from the tissue exhibiting the gene-silencing-construct-associated phenotype, particularly the necrotized tissue.

It is yet another object of the invention to provide a method for introduction of inhibitory RNA, preferably sense or antisense RNA, particularly inhibitory RNA comprising a complementary stretch of at least 50, preferably at least 100 nucleotides of sense and antisense RNA, into plant cells, preferably into the cytoplasm of plant cells, comprising introducing into a plant cell, a viral RNA vector comprising the inhibitory RNA or comprising a chimeric nucleic acid which when transcribed yields the inhibitory RNA, wherein the inhibitory RNA comprises a sense RNA or an antisense RNA comprising a nucleotide sequence of at least 100 nucleotides in length having at least 75% sequence identity to the nucleotide sequence of the target gene in the plant cell, and wherein the viral RNA vector comprises at least cis elements from a satellite RNA virus, preferably STMV or STNV, particularly a STMV - derived or STNV derived RNA vector comprising an origin of assembly from tobacco mosaic virus, recognized by a replicase from a corresponding helper virus and is capable of replicating in a plant cell only when provided externally with essential functions required for said replication; and introducing into the same plant cell, a corresponding helper virus, preferably tobacco mosaic virus or tobacco necrosis virus comprising a coat protein gene of tobacco mosaic virus

The invention further provides a kit for introduction of inhibitory RNA, preferably sense or antisense RNA, particularly inhibitory RNA comprising a complementary stretch of at least 50, preferably at least 100 nucleotides of sense and antisense RNA in the cytoplasm of a plant cell comprising 1) a viral RNA vector comprising the inhibitory RNA or a chimeric nucleic acid which when transcribed yields the inhibitory RNA, wherein the inhibitory RNA comprises a sense RNA or an antisense RNA comprising a nucleotide sequence of at least 100 nucleotides in length having at least 75% sequence identity to the nucleotide sequence of the target gene in the plant cell, and wherein the viral RNA vector comprises at least cis elements from a satellite RNA virus recognized by a replicase from a corresponding helper virus and is capable of replicating in a plant cell only when provided externally with essential functions required for said replication; and 2) a corresponding helper virus.

A particularly preferred kit comprises 1) a viral RNA vector comprising at least cis elements from a satellite tobacco mosaic virus, further comprising or encoding the inhibitory RNA; and 2) a corresponding helper virus which is a tobacco mosaic virus.

Another particularly preferred kit comprises 1) a viral RNA vector comprising at least cis elements from satellite tobacco necrosis virus, especially STNV-2 or STNV-C, and further comprising an origin of assembly of tobacco mosaic virus, further comprising or encoding the inhibitory RNA; and 2) a corresponding helper virus which is tobacco necrosis virus, particularly TNV-A or TNV-D, which comprises a coat protein gene of tobacco mosaic virus.

### Detailed description of preferred embodiments.

The following definitions apply throughout this application, unless otherwise specified.

As used herein "comprising" is to be interpreted as specifying the presence of the stated features, integers, steps or components as referred to, but does not preclude the presence or addition of one or more features, integers, steps or components, or groups thereof. Thus, e.g., a nucleic acid or protein comprising a sequence of nucleotides or amino acids, may comprise more nucleotides or amino acids than the actually cited ones, i.e., be embedded in a larger nucleic acid or protein. A chimeric gene comprising a DNA region which is functionally or structurally defined, may comprise additional DNA regions etc.

As used herein, "a trait of a plant" indicates a phenotype which is the combined result of the coordinated expression of a number of genes. Typical traits include yield, heterosis, drought-resistance, stress-resistance, high or low temperature-resistance, vigor, seed yield, plant habitat, architecture etc. Typically, a trait of a plant is named after its intended appearance.

A used herein a "phenotype" of a plant refers to any quantitative or qualitative characteristic of that plant, be it morphological (including macroscopic and microscopic characteristics), biochemical (including the presence, absence or concentration of particular metabolites or molecules) functional or other.

The term "gene" means any DNA or RNA fragment comprising a region (the "transcribed region") which is transcribed into a RNA molecule (e.g., a mRNA) in a cell, operably linked to suitable regulatory regions, e.g., a plant-expressible promoter. A gene may thus comprise several operably linked fragments such as a promoter, a 5' leader sequence, a coding region, and a 3' region comprising a polyadenylation site. A plant gene endogenous to a particular plant species or virus (endogenous plant or virus gene) is a gene which is naturally found in that plant species or virus, or which can be introduced in that plant species by breeding techniques such as conventional breeding techniques. A chimeric gene is any gene which is not normally found in a plant species or, alternatively, any gene in which the promoter is not associated in nature with part or all of the transcribed DNA region or with at least one other regulatory region of the gene.

The term "expression of a gene" refers to the process wherein a DNA or RNA region which is operably linked to appropriate regulatory regions, particularly to a promoter, is transcribed into an RNA which is biologically active i.e., which is either capable of interaction with another nucleic acid or which is capable of being translated into a biologically active polypeptide or protein. A gene is said to encode an RNA when the end product of the expression of the gene is biologically active RNA, such as e.g. an antisense RNA, a ribozyme or a replicative intermediate. A gene is said to encode a protein when the end product of the expression of the gene is a biologically active protein or polypeptide. In addition to the above defined elements, a gene may further comprise elements for cap-independent translation such a an internal ribosome entry sequence or the first and second translation enhancing elements as defined in WO 97/49814.

As used herein the terms "gene-silencing" or "inhibitory" are not to be interpreted as meaning a complete abolishing of the expression of the target gene(s) but also includes any reduction in expression, measured either as a reduction in transcription and/or translation, as a reduction in the accumulation of transcripts or translation products such as proteins, or as a reduction in the phenotypic expression of the target gene.

The term "reduction of phenotypic expression" refers to the comparison of the phenotypic expression of the nucleic acid of interest in the eukaryotic cell in the presence of the inhibitory RNA or gene-silencing constructs of the invention, to the phenotypic expression of the nucleic acid of interest in a similar eukaryotic cell in the absence of the inhibitory RNA or gene-silencing constructs of the invention. The phenotypic expression in the presence of the inhibitory RNA of the invention should thus be lower than the phenotypic expression in absence thereof, preferably be only about 25%, particularly only about 10%, more particularly only about 5% of the phenotypic expression in absence of the inhibitory RNA, especially the phenotypic expression should be completely inhibited for all practical purposes by the presence of the inhibitory RNA or the gene-silencing construct encoding such an RNA.

A reduction of phenotypic expression of a nucleic acid where the phenotype is a qualitative trait means that in the presence of the inhibitory RNA, the phenotypic trait switches to a different discrete state when compared to a situation in which such inhibitory RNA is absent. A reduction of phenotypic expression of a nucleic acid may thus a.o. be measured as a reduction in transcription of (part of) that nucleic acid or reduction in the level of transcript, a reduction in translation of (part of ) that nucleic acid or reduction in the level of translation products, or a reduction of the effect the presence of the transcribed RNA(s) or translated polypeptide(s) have on the eucaryotic cell or the organism, and will ultimately lead to altered phenotypes. It is clear that the reduction in phenotypic expression of a nucleic acid of interest, may be accompanied by or correlated to an increase in a phenotype or trait.

In one embodiment of the invention, a method is provided to identify and isolate genes involved in the determination of a trait or a phenotype of a plant. To this end, nucleic acid sequences are identified whose expression is correlated with the trait and/or phenotype of interest. Methods and means are available in the art for the almost simultaneous identification and/or isolation of a large number, if not the predominant part, of nucleotide sequences whose expression, particularly whose transcription, is influenced subsequent to a stimulus corresponding to the trait to be investigated, in comparison with expression of these nucleotide sequences in a control plant. Such methods include but are not limited to differential display methods, such as the gel - based RNA differential display methods described by (Prahasar et al. 1996), As a result of these methods, a collection of at least partially characterized nucleotide sequences with altered expression in response to a particular stimulus is identified. Typically, however, the application of such methods does not allow to discriminate between genes whose altered expression is directly caused by the stimulus, and those who are further downstream in the chain of events and are only indirectly influenced by the stimulus and a further selection amongst the obtained collection of nucleotide sequences will be required. Even less do these methods allow to predict whether the inverse relationship also holds, i.e. whether influencing the expression of genes with particular nucleotide sequences, identified in the above mentioned way, also influences the trait of interest. A further validation of the obtained sequences is thus required, and preferably one which immediately verifies the above mentioned inverse relationship. To achieve this goal in a efficient and cost-effective way, a library of gene-silencing constructs may be created in a viral RNA vector which is capable of replication inside plant cells. The created library of gene-silencing constructs comprised within a viral RNA vector is then used to infect a representative number of plants in such a way that at each plant is infected by at least one member (one clone) of the library. The infected plants can than be analyzed to identify those plants which exhibit alterations in the trait under investigation, using an assay which is adapted to the trait under investigation. It is clear that this fine-tuning of assay and trait under investigation may be an important advantage over the existing methods for high throughput analysis, particularly when analyzing traits and/or phenotypes which do not result in microscopically visible alterations, such as but not limited to modifications in specific metabolic pathways or alterations which are only detectable under specific conditions (e.g. heat, stress, drought-tolerance, pathogen-infection, application of specific herbicides or insecticides etc.).

The subset of nucleic acid sequences may also be identified on the basis of the presence of a particular signature characteristic of a class of proteins, such as but not limited to a kinase-specific domain, a binding motif etc.

The gene-silencing construct may then be isolated from the library or from the plant exhibiting the altered trait or phenotype, and be used to isolate the corresponding gene based on the nucleotide sequence towards which the gene silencing construct was targeted.

The inventors have obtained for the first time indications that gene-silencing may be obtained in plant cells such as protoplasts, using a viral vector derived from a satellite virus comprising a β-1,3-glucanase coding region, in a co-infection experiment with a helper virus of the satellite virus

In another preferred embodiment, the viral RNA vector is capable of replication, only when the required functions are provided *in trans.* Particularly preferred, is the use of a satellite virus derived RNA vector, which can replicate in plant cells and spread throughout the plant, when a corresponding helper virus is present.

As used herein, "a satellite virus" indicates an RNA virus, preferably a single stranded RNA virus, the RNA genome of which is capable of replicating in a plant cell and being encapsidated by coat protein molecules to form a virus particle or vision, only when provided externally with any number of required essential functions therefor. By "externally provided" is meant that such functions are not encoded by the satellite viral genome. Satellite viruses thus depend upon external provision of essential functions, and may lack the capacity to encode functional replicase, movement protein, or other essential functions required to complete their life cycle inside a plant cell. In a natural situation, such essential functions are usually provided by an autonomously replicating virus or so-called helper virus.

Satellite viruses useful for the present invention may include wild type isolates, but also encompassed by this definition are variants which result in reduced or minimal symptoms when infected on a host plant, particularly when co-inoculated with a corresponding helper virus. The definition also includes synthetic satellite viruses such as defective viruses and chimeric satellite viruses.

A "viral RNA vector derived from a satellite virus" should at least include cis elements from a satellite virus which are recognized by an externally provided replicase, and an origin of assembly allowing encapsidation by the provided coat protein. Preferably, the viral RNA vector does not comprise a gene encoding a functional coat protein, particularly it does not comprise the nucleotide sequence which is essentially similar to the nucleotide sequence encoding a coat protein gene. Particularly preferred are viral RNA vectors comprising an origin of assembly recognized by coat protein molecules from a rod-shaped virus, such as tobacco mosaic virus, since rod-shaped viruses do not exhibit the spatial constraints imposed on the size of genome by icosahedral viruses, thus allowing a larger number of additional nucleotides to be incorporated in the viral vector. Conveniently, the viral RNA vector comprises a number of unique or low-occurrence restriction recognition sites.

The use of viral RNA vectors derived from a satellite virus, additionally solves problems associated with the use of viral RNA vectors, such as reducing the size of the vectors, increasing versatility etc.

Particularly suited for the invention are viral RNA vectors derived from satellite tobacco mosaic virus comprising the origin of assembly (OAS) from TMV, preferably comprising the nucleotide sequence of SEQ ID No 2 from the nucleotide at position 5443 to the nucleotide at position 5518 or the nucleotide of SEQ ID No 5 from the nucleotide at position 5430 to the nucleotide at position 5505 (such as the nucleotide sequence of SEQ ID No 12) and wherein the coat protein encoding gene has been deleted. Also particularly suited for the invention are viral RNA vectors derived from satellite necrosis vector strain comprising the OAS from TMV and wherein most of the coat protein gene has been deleted. Non-limiting examples of viral RNA vectors, suitable for the invention are described hereinafter.

"A corresponding helper virus" as used herein, indicates those RNA viruses, preferably singe stranded RNA viruses, which can supply the satellite virus or the derived viral RNA vector with the functions required *in trans* by that satellite virus or the derived viral RNA vector, to allow it to replicate in the cytoplasm of plant cells, and spread throughout an infected plant. Typically, corresponding helper viruses will provide the satellite virus or the vector derived thereof with a replicase (RNA dependent RNA polymerase) which recognizes the *cis* sequences present on the satellite virus RNA, and will allow replication of the satellite virus genome or the derived vector. Other proteins which may typically be provided by the helper virus are movement proteins, allowing inter alia, the plasmodesmata-mediated spread of viral particles from cell to cell. For satellite viruses or viral RNA vectors derived thereof which lack a functional coat protein encoding gene, corresponding helper viruses may also provide a functional coat protein. Preferably, the corresponding helper virus will be capable of autonomous systemic spread in an infected plant. However, such a systemic spread seems not to be a prerequisite for efficient gene silencing. Functions required *in trans* for one particular viral RNA vector may be supplied *in trans* by different corresponding helper viruses.

It is clear that the corresponding helper viruses may be wild type isolates of RNA viruses, preferably single-stranded RNA viruses such as the tobamoviruses or necroviruses. Particularly preferred are rod-shaped RNA viruses such as tobamoviruses including tobacco mosaic virus and the related tobamoviruses such as ribgrass mosaic virus, turnip vein clearing virus, chines rape mosaic virus, oilseed rape mosaic virus.

When TMV can be used as a helper virus, it can also be replaced by one of the closely related tobamoviruses mentioned above, particularly when using the viral vectors in particular plant species.

Also encompassed by the methods and means of the invention are variants of such wild type isolates, preferably variants or mutants which develop minimal symptoms when inoculated on host plants or when co-infected with a corresponding satellite virus or RNA vector derived thereof. Further preferred helper viruses may be variants or mutants of wild type isolates which have an extended host range such as tobamoviruses which can replicate and spread in corn or brassicae.

However, corresponding helper viruses may also be chimeric or hybrid viruses, wherein part of the viral genome has been replaced by a foreign nucleic acid, particularly wherein part of the viral genome has been replaced by a nucleic acid derived from another viral genome, preferably a part comprising a nucleotide sequence encoding a movement protein, or a part comprising a nucleotide sequence encoding a coat protein. E.g. when using a necrovirus such as TNV, it may be advantageous to insert a movement protein encoding region , preferably a movement protein derived from a tobamovirus such as TMV, particularly the nucleotide sequence of SEQ ID No 2 from the nucleotide at position 4903 to the nucleotide at position 5709, so as to ensure spreading of the viral particles beyond the infected leaf. However, spreading of the helper virus or the viral RNA vector is not essential for efficient inactivation of expression of the target genes throughout the plant, as was found by the inventors. Also when using e.g. a necrovirus such as TNV, it may be further advantageous to replace the coat protein coding region of the necrovirus by a coat protein coding region of a rod-shaped virus, such as TMV, particularly the nucleotide sequence of SEQ ID No 2 from the nucleotide at position 5712 to the nucleotide at position 6191. It goes without saying that an appropriate origin of assembly for the substituted coat protein has to be incorporated in the genome of the chimaeric helper virus. In the above described example however, the OAS of TMV is conveniently located within the movement protein coding region. Non-limiting examples of corresponding helper viruses will be described hereinafter.

It should be clear that whenever it is stated that plants are co-infected or infected with a viral RNA vector and a corresponding helper virus, it is equal whether the helper virus is inoculated before, after or simultaneous with the viral RNA vector, provided however that there is a reasonable time limit between infection of the viral RNA vector or the corresponding helper virus.

Alternatively, the required functions *in trans* for the replication and movement of the viral RNA vector may be provided from the expression of chimeric genes, encoding a replicase (RNA dependent RNA polymerase) and/or a movement protein and/or a functional coat protein, integrated in the genome of the test plants.

Preferred kits to deliver inhibitory RNA or gene-silencing constructs to plant cells to be used in the herein disclosed methods comprise a viral RNA vector derived from a satellite RNA virus, particularly from satellite tobacco necrosis vector (STNV) or satellite tobacco mosaic virus (STMV) and a corresponding helper virus, particularly a rod-shaped corresponding helper virus, wherein the viral RNA vector comprises a gene-silencing construct.

In a preferred embodiment the kit comprises a viral RNA vector derived from satellite tobacco necrosis vector, preferably comprising the cis-elements required for replication, particularly comprising the nucleotide sequence of SEQ ID No 3 from the nucleotide at position 1 to the nucleotide at position 32 and the nucleotide sequence of SEQ ID No 3 from the nucleotide at position 738 to the nucleotide at position 1245, wherein an origin of assembly of tobacco mosaic virus has been inserted, preferably comprising the nucleotide sequence of SEQ ID No 2 from the nucleotide at position 5443 to the nucleotide at position 5518 or comprising the nucleotide sequence of SEQ ID No 5 from the nucleotide at position 5430 to the nucleotide at position 5505, or comprising the nucleotide sequence of SEQ ID No 12 and wherein said helper virus is derived from tobacco necrosis virus, preferably with a nucleotide sequence of SEQ ID No 1, and comprises a gene encoding the movement protein of tobacco mosaic virus, preferably with the nucleotide sequence of SEQ ID No 2 from the nucleotide at position 4903 to the nucleotide at position 5709 or with the nucleotide sequence of SEQ ID No 15 from the nucleotide at position 479 to the nucleotide at position 1285 and a gene encoding the coat protein of tobacco mosaic virus, preferably with the nucleotide sequence of SEQ ID No 2 from the nucleotide at position 5712 to the nucleotide at position 6191 or with the nucleotide sequence of SEQ ID No 15 from the nucleotide at position 1288 to the nucleotide at position 1767.

Preferred combinations are those kits wherein the viral RNA vector is derived from STNV-1 or STNV-2 strains (as disclosed by Ysebaert et al. 1980; Genbank Accession number M10388 or Danthinne et al., 1991 Genbank Accession M64479) and the helper virus is TNV-A (Meulewaeter et al 1990, SEQ ID No 1). Other preferred combinations are those kits wherein the viral RNA vector is derived from STNV-C (Bringloe et al. (1998); Genbank Accession Nr AJ000898) and the corresponding helper virus is TNV-D (Coutts et al. (1991); Genbank Accession Nr D00942).

In another Particularly preferred embodiment the kit comprises a viral RNA vector derived from satellite tobacco mosaic virus, preferably comprising the cis-elements required for replication, particularly comprising the nucleotide sequence of SEQ ID No 4 from the nucleotide at position 1 to the nucleotide at position 197 and the nucleotide sequence of SEQ ID No 4 from the nucleotide at position 604 to the nucleotide at position 1058 or comprising the nucleotide sequence of SEQ ID No 13 and the nucleotide sequence of SEQ ID No 14; and further comprising an origin of assembly of tobacco mosaic virus, preferably comprising the nucleotide sequence of SEQ ID No 2 from the nucleotide at position 5443 to the nucleotide at position 5518 or comprising the nucleotide sequence of SEQ ID No 5 from the nucleotide at position 5430 to the nucleotide at position 5505 or comprising the nucleotide sequence from SEQ ID No 12, and wherein said corresponding helper virus is a tobacco mosaic virus, particularly TMV-U1 (SEQ ID No 2) or TMV-U2 (SEQ ID No 5).

It will be clear to the person skilled in the art that viral RNA vectors may be generated conveniently by *in vitro* transcription methods from cDNA copies of the viral RNA. Likewise, infectious viral RNA for the corresponding helper viruses may be generated from cDNA copies of their genome. Libraries, viral vectors and corresponding helper viruses may also be maintained by replication in plant cells.

Methods to infect or inoculate plants and plant cells with viral RNA vectors, helper viruses and libraries comprised within viral RNA vectors are well within in the realm of the person skilled in the art and may be performed according to the methods described in Walkey (1985).

In one embodiment of the methods of the invention, plants are inoculated, e.g. with a solution containing the libraries of gene-silencing constructs in a viral vector, or with a solution containing a mixture of gene-silencing constructs in a viral vector and corresponding helper virus. The solution may further contain additional compounds to improve inoculation and infection of the plants, such as, but not limited to abrasives, adherents, tensio-active products and the like.

Plants may be infected during different developmental stages, in order to maximize the phenotype under investigation. Also different parts of plants may be inoculated to optimize observation of the expected phenotype.

Although not intending to limit the scope of the invention to a particular mode of action, it is thought that the inhibitory RNA comprised within the viral RNA vector can exercise its inhibiting effect, provided there is a balance between RNA encapsidated in a virion and free RNA. It is thought that the balance between encapsidated and free RNA may be influenced by varying the sequence and position of an origin of assembly within the viral RNA vector. However, the gene-silencing effect may be amplified by placing the inhibitory RNA encoding nucleic acid under control of a viral promoter, preferably a coat protein promoter, or a subgenomic promoter so that during the life cycle of the virus additional inhibitory RNA is generated or transcribed.

"Gene-silencing constructs" as used herein is to be interpreted as a nucleic acid, which when transcribed yield "inhibitory RNA" comprising or consisting of sense RNA or antisense RNA, or a combination of both comprising a nucleotide sequence which has at least 75%, preferably at least 80%, particularly at least 85%, more particularly at least 90%, especially at least 95% sequence identity with or is identical to the nucleotide sequence whose expression is to be suppressed, or its complement. Further, the nucleotide sequence of the sense or antisense region should preferably be at least about 100 nucleotides in length, more preferably at least about 250 nucleotides, particularly at least about 500 nucleotides but may extend to the full length of the coding region of the gene whose expression is to be reduced.

For practical purposes in the application of the methods for high throughput screening or validation, the gene-silencing construct may be identical in sequence and length to the target nucleic acids, or they may be exactly complementary in sequence and identical in length to the target nucleic acids.

For the purpose of this invention the "sequence identity" of two related nucleotide or amino acid sequences, expressed as a percentage, refers to the number of positions in the two optimally aligned sequences which have identical residues (x100) divided by the number of positions compared. A gap, i.e. a position in an alignment where a residue is present in one sequence but not in the other is regarded as a position with non-identical residues. The alignment of the two sequences is performed by the Wilbur and Lipmann algorithm (Wilbur and Lipmann ,1983) using a window-size of 20 nucleotides or amino acids, a word length of 2 amino acids, and a gap penalty of 4. Computer-assisted analysis and interpretation of sequence data, including sequence alignment as described above, can be conveniently performed using commercially available software packages such as the programs of the Intelligenetics^{™} Suite (Intelligenetics Inc., CA) or the GCG Wisconsin Package.

It is clear for the person skilled in the art that the gene-silencing constructs may comprise at the same time sense and anti-sense RNA targeted towards the same nucleotide sequence whose expression is to be reduced. Preferably, the sense and antisense RNA are at least partly complementary to each other and capable of forming a stem-loop structure, since such a configuration has been shown to increase the efficiency of gene-silencing, both in occurrence and level of gene-silencing (Waterhouse et al. 1998). In the most straightforward embodiment, at least part of the target nucleic acid, preferably the complete target nucleic acid, is cloned in duplicated form, whereby the two copies are in inverted repeat, preferably separated by an unrelated spacer nucleotide sequence.

The invention also aims at providing the herein described kits in their different embodiments. It is also an object of the invention to provide the kits comprising the helper viruses and viral RNA vectors described without the gene-silencing constructs or inhibitory RNA as well as their cDNA copies, whereby the cDNA copies are under control of a promoter (which can be used in *in vitro* transcription methods available in the art) such as but not limited to the promoters recognized by single subunit bacteriophage polymerase promoters (T7, T3, SP6 RNA polymerase specific promoters and the like).

The invention further relates to a method for identifying genes which are essential in plants comprising the following steps:
a) A library of random gene-silencing constructs specific for the plant is created using a viral RNA vector which is derived from a satellite RNA virus, as herein defined including all its preferred embodiments. Preferably, the library is created in a cDNA copy of the viral vector and may be generated by inserting random DNA sequences, preferably at least about 100 nucleotides in length, particularly at least about 500 nucleotides in length. The random DNA sequences may be obtained from total DNA of a plant or may represent a subset of the genome of a plant, such as DNA derived from organelles (plastids, chloroplasts, mitochondria etc.) Alternatively, the library may be created by inserting cDNAs generated by reverse transcriptase from RNA, preferably mRNA obtained from said plant. The library may be normalized, e.g. as described in Takayuki et al. (1995). The library should preferably be large enough in size, i.e. contain a sufficient number of independent clones to cover the genome of the plant, according to the standards known in the art. In a preferred embodiment, the library may contain duplication of the inserted nucleic acid whereby the copies are in inverted repeat. The inserted nucleic acid may be cloned downstream of a viral promoter such as, but not limited to a coat protein gene promoter or a subgenomic promoter. It will further be clear to the person skilled in the art that the relative orientation of the inserted nucleic acid, in relation to the RNA vector is only of limited importance since either sense or antisense inhibitory RNA will be generated.
b) Assay plants are infected with individual members of the library and also with a corresponding helper virus. Infection may proceed according to any of the methods mentioned herein. Clearly, the DNA copy of the library should be converted into an RNA copy according to any of the methods described herein, preferably prior to the infection of the assay plants.
c) Plants developing a gene-silencing-construct-associated phenotype are identified. As used herein, a "gene-silencing-construct-associated phenotype" is meant to indicate a phenotype which is not observed when performing a mock inoculation with a viral RNA vector without gene-silencing construct, in combination with a corresponding helper virus on a similar plant. Preferred phenotypes comprise chlorosis, necrosis or any phenotype, preferably a morphological phenotype indicating that the infected tissue is inhibited or dying or deteriorating.
d) Optionally , isolating the viral RNA vector from the tissue exhibiting the gene-silencing-construct-associated phenotype according to methods available in the art for isolation of virus. Preferably, the isolated viral RNA vector comprising the gene-silencing construct of interest should be re-assayed on fresh plants to confirm the observed phenotype. The viral RNA vector can of course also be recovered from the library if the infection of the plants was performed in an identity -preserving way.
e) The gene silencing construct or the nucleotide sequence information thereof, may then be used to recover the corresponding genomic or cDNA clone using methods available in the art (hybridization, PCR etc.)

As defined herein "essential genes" of a plant, are those genes which are necessary during the normal development of a plant. As defined, essential genes may be essential for normal development only in particular developmental stages, or only in particular tissues or organs, such as e.g. flowers. Typically, inhibition of the expression of essential genes may have a lethal effect on a plant or part of a plant. Preferred essential genes are those genes which result in retardation or dying of seedlings when inhibited.

It will be clear for the person skilled in the art that if the inhibition of the target nucleic acid results in a dominant effect, as is the case for inhibition of the expression of essential genes, the described methods may be performed using infection of more than one viral RNA vector comprising a gene-silencing construct per plant. Care has to be taken to not dilute the phenotypic effect too much by infecting a too large number of different viral RNA vectors comprising differing gene-silencing constructs on the same plant. It is thought that optimally any number between one and five different inhibitory RNAs may be introduced in one plant cell.

In yet another embodiment of the invention, a method is provided for determining the function encoded by a nucleic acid comprising a known nucleotide sequence. This nucleotide sequence may have been obtained e.g. from a genome sequencing program, including expressed sequence tags sequencing programs. In order to unravel the function of that sequence, a gene-silencing construct or inhibitory RNA targeted towards said nucleotide sequence, as described in all its embodiments herein, may be introduced into a viral RNA vector derived from a satellite virus, as described herein, and used to inoculate a plant or being introduced into a plant cell, particularly into a protoplast, together with a corresponding helper virus, as described herein.

A large number of the embodiments described herein thus relate to a method for the introduction of inhibitory RNA in plant cells, comprising the steps of :
a.) introducing into a plant cell, a viral RNA vector comprising inhibitory RNA or comprising a chimeric nucleic acid which when transcribed yields the inhibitory RNA, wherein the viral RNA vector is derived from a satellite RNA virus; and
b.) introducing into the same plant cell, a corresponding helper virus.

The methods of the invention can be applied to essentially all plants for which viral vector and/or corresponding helper viruses are available. The methods of the invention are thought to be particularly suited for Nicotina spp, particularly N. tabacum, N. sylvestris, N. benthamiana, and other Solanacea, rice (Oryza sativa) corn (Zea Mays), Brassica spp. , cotton (Gossypum hirsutum), wheat. Arabidopsis spp., Petunia spp.

Also envisioned by the present invention are methods for developing an agronomically useful product, such as a herbicide or a transgenic plant using the herein described methods and means, further comprising the steps of inserting a nucleic acid, involved in the determination of a particular plant trait, isolated by the methods of the invention, preferably under control of a foreign plant-expressible promoter, particularly under control of a controllable plant-expressible promoter into the genome of a plant , particularly a crop plant. When essential genes have been identified according to the methods described herein, these essential genes or their encoded gene products, particularly the encoded proteins may be used in *in vitro* assays to identify compounds inhibiting the activity, particularly the enzymatic activity, which may be used as herbicides. Alternatively, a viral RNA vector encoding gene-silencing constructs targeted towards essential genes may be used as herbicidal compounds.

The following non-limiting Examples describe the construction of viral RNA vectors derived from satellite viruses, and uses thereof. Unless stated otherwise in the Examples, all recombinant DNA techniques are carried out according to standard protocols as described in Sambrook et al. (1989) Molecular Cloning: A Laboratory Manual, Second Edition, Cold Spring Harbor Laboratory Press, NY and in Volumes 1 and 2 of Ausubel et al. (1994) Current Protocols in Molecular Biology, Current Protocols, USA. Standard materials and methods for plant molecular work are described in Plant Molecular Biology Labfax (1993) by R.D.D. Croy, jointly published by BIOS Scientific Publications Ltd (UK) and Blackwell Scientific Publications, UK.

Throughout the description and Examples, reference is made to the following sequences:
- SEQ ID No 1:: nucleotide sequence of the genome of TNV-A
- SEQ ID No 2:: nucleotide sequence of the genome of TMV-U1 ( Genbank Accession Nr V01408).
- SEQ ID No 3:: nucleotide sequence of the genome of STNV-2
- SEQ ID No 4:: nucleotide sequence of the genome of STMV (Genbank accession Nr. M25782).
- SEQ ID No 5:: nucleotide sequence of the genome of TMV-U2 ( Genbank Accession Nr M34077).
- SEQ ID No 6:: nucleotide sequence of the tomato phytoene desaturase (*pds*) encoding cDNA (Genbank Accession Nr. X59948).
- SEQ ID No 7:: nucleotide sequence of the tobacco nitrate reductase (*nia*-2) encoding cDNA (Genbank Accession Nr. X14059).
- SEQ ID No 8:: nucleotide sequence of the tobacco nitrite reductase (*nir*-1) encoding cDNA (Genbank Accession Nr. X66145).
- SEQ ID No 9:: nucleotide sequence of the β-1,3-glucanase( *gn*-1) encoding cDNA of *Nicotiana plumbagenifolia*.
- SEQ ID No 10:: nucleotide sequence of a green fluorescent protein (*gfp*) encoding region.
- SEQ ID No 11:: nucleotide sequence of a β-glucuronidase (*gus*) encoding region.
- SEQ ID No 12:: nucleotide sequence of an origin of assembly of a TMV-U2 strain.
- SEQ ID No 13:: nucleotide sequence of the leader sequence of a STMV strain
- SEQ ID No 14:: nucleotide sequence of the trailer sequence of a STMV strain.
- SEQ ID No 15:: nucleotide sequence of part of the genome of a TMV-U2 strain comprising movement protein and coat protein genes.

### Examples

### Example I : construction of the viral RNA vector kits

A plasmid vector for the synthesis of an infective hybrid TMV/TNV helper virus RNA is made using the following operationally linked elements:
- A T7 RNA polymerase promoter
- A nucleic acid comprising the nucleotide sequence from the nucleotides 1 to 2234 of TNV-A (nt 1 to 2234 of SEQ ID No 1), wherein the AUG codon at nucleotides 2218-2220 mutated to a different codon
- A nucleic acid comprising the nucleotide sequence encoding the open reading frame for the movement protein of TMV-U1 (nt 4903-5709 of Genbank Accession Number V01408 or nt 4903-5709 of SEQ ID No 2 or nt 479-1285 of SEQ ID No 12)
- A nucleic acid comprising the nucleotide sequence from the nucleotides 2235 to 2612 of TNV-A (SEQ ID No 1)
- A nucleic acid comprising the nucleotide sequence encoding the open reading frame for the coat protein of TMV-U1 (nt 5712-6191 of Genbank Accession Number V01408 or nt 5712-6191 of SEQ ID No 2 or nt 1288-1767 of SEQ ID No 15)
- A sequence comprising nucleotide 3444 to 3684 of TNV-A (nt 3444 to 3684 of SEQ ID No 1)

A plasmid vector for the synthesis of an infective hybrid TMV/STNV viral vector RNA is made using the following operationally linked elements:
- A T7 RNA polymerase promoter
- A nucleic acid comprising the nucleotide sequence from nucleotide 1 to 32 of STNV-2 (nt 1 to 32 of SEQ ID No 3)
- A nucleic acid comprising the origin of assembly (OAS) of TMV-U1 (nt. 5443-5518 of Genbank Accession Number V01408 or nt 5443-5518 of SEQ ID No 2 or nt 1018 to 1094 of SEQ ID No 15)
- A nucleic acid comprising the nucleotide sequence from nucleotide 738 to 1245 of STNV-2 (nt 738 to 1245 of SEQ ID No 3)

A plasmid vector for the synthesis of an infective hybrid TMV/STMV viral vector RNA is made using the following operationally linked elements:
- A T7 RNA polymerase promoter
- A nucleic acid comprising the nucleotide sequence from nucleotide 1 to 197 of STMV (Genbank Accession Number M25782; nt 1 to 197 of SEQ ID No 4) or the nucleotide sequence of SEQ ID No 13.
- A nucleic acid comprising the OAS of TMV-U1 (nt. 5443-5518 of Genbank Accession Number V01408; nt 5443 to 5518 of SEQ ID No 2; nt 1019-1094 of SEQ ID No 15) or of TMV-U2 (nt 5430-5505 of Genbank Accession Number M34077; nt 5430-5505 of SEQ ID No 5) such as the nucleotide sequence of SEQ ID No 12.
- A nucleic acid comprising the nucleotide sequence from nucleotide 604 to 1058 of STMV (Genbank Accession Number M25782; nt 604 to 1058 of SEQ ID No 4) or comprising the nucleotide sequence of SEQ ID No 14.

### Example 2. Feasibility demonstration using known endogenes or transgenes.

To demonstrate the feasibility of the use of the viral kits described sub example 1 for functional knockout of specific endo- or transgenes in *Nicotiana* plants, one of the following DNA fragments is inserted in the TMV/STNV or TMV/STMV hybrid vector, immediately upstream or downstream of the TMV OAS:
- a fragment of the tomato pythoene desaturase (*pds*) cDNA (comprising nucleotide 1021 to 1671 of SEQ ID No 6 or Genbank Accession Number X59948)
- a fragment of the tobacco nitrate reductase (*nia-2*) cDNA (comprising nucleotides 1103 to 2114 or nucleotides 5169 to 6497 of SEQ ID No 7 or Genbank Accession Number X14059)
- a fragment of the tobacco nitrite reductase (*nir-1*) cDNA (comprising nucleotide 650-1212 of SEQ ID No 8 or Genbank Accession Number X66145)
- a fragment of the β-1,3-glucanase (*gn-1*) cDNA of *Nicotiana plumbaginifolia* (SEQ ID No 9 or Genbank Accession Number X07280)
- a fragment comprising a nucleotide sequence from a green fluorescent protein (*gfp*) coding region (SEQ ID No 10)
- a fragment comprising a nucleotide sequence from nucleotide 1 to 600 of the β-glucuronidase (*gus*) coding region (SEQ ID No 11)

Infective chimeric transcripts are synthesized *in vitro,* using T7 RNA polymerase with the linearized plasmid DNAs of the described vectors as templates.
The TMV/STNV RNAs are mechanically inoculated on leaves of *Nicotiana benthamiana* or *Nicotiana tabacum* plants together with the TMV/TNV RNA, whereas the TMV/STMV RNAs are inoculated together with TMV-U2 virus particles or viral RNA.
The infected plants are scored for phenotypes, virus accumulation, and suppression of the homologous plant gene between 1 and 4 weeks after inoculation.
Plants infected with vectors containing the pds cDNA show a bleaching phenotype on infected leaves and silencing of the endogenous pds transcript.
Plants infected with vectors containing the *nia-2* or *nir-1* cDNA show a chlorotic phenotype on infected leaves and silencing of the endogenous *nia-2* or *nir-1* transcript, respectively.
Plants infected with vectors containing the g*n-1* cDNA show silencing of the endogenous basic -1,3-glucanase transcript.
Upon infection with vectors containing the *gfp* sequence, transgenic plants that normally express a *gfp* transgene show silencing of the *gfp* transgene transcript and suppression of GFP fluorescence.
Upon infection with vectors containing the *gus* sequence, transgenic plants that normally express a *gus* transgene show silencing of the *gus* transgene transcript and suppression of GUS activity.

### Example 3: Inactivation of phytoene desaturase in Nicotiana benthamiana using a TNV/STNV hybrid vector system.

A TNV/STNV hybrid vector system was used for the functional inactivation of a constitutively expressed endogenous plant gene . Therefore, the following STNV hybrid vectors have been constructed:
pIF9 carrying the following operationally linked elements:
   - a T7 RNA polymerase promoter comprising nucleotide 402 to 420 of Genbank Accession Number M77811;
   - a nucleic acid comprising the nucleotide sequence from nucleotide 1 to 32 of SEQ ID No 3 (STNV-2 leader);
   - a nucleic acid comprising the origin of assembly (OAS) of TMV-U1 from nucleotide 5443 to 5518 of SEQ ID No 2 or Genbank Accession Number V01408;
   - a fragment of the tomato phytoene desaturase (pds) cDNA comprising nucleotide 1021 to 1671 of SEQ ID No 6 or Genbank Accession Number X59948;
   - a nucleic acid comprising the nucleotide sequence from nucleotide 806 to 1418 of SEQ ID No 3 (STNV-2 trailer).
pIF12 carrying the following operationally linked elements:
   - a T7 RNA polymerase promoter comprising nucleotide 402 to 420 of Genbank Accession Number M77811;
   - a nucleic acid comprising the nucleotide sequence from nucleotide 1 to 32 of SEQ ID No 3 (STNV-2 leader);
   - a fragment of the tomato phytoene desaturase (pds) cDNA comprising nucleotide 1021 to 1671 of SEQ ID No 6 or Genbank Accession Number X59948;
   - a nucleic acid comprising the nucleotide sequence from nucleotide 742 to 1354 of SEQ ID No 3 (STNV-2 trailer).

Infective chimeric transcripts have been synthesized in vitro using T7 RNA polymerase with the linearized plasmid DNAs of the described pIF9 and pIF12 hybrid vectors as templates using standard procedures. Control in vitro transcripts have been synthesized on linearized plasmid DNAs of the precursor plasmids of pIF9 and pIF12 without the pds fragment inserts and on linearized plasmid DNA of an infective clone of the STNV wild type and of a hybrid STNV vector carrying an insert of the cat gene.

The in-vitro transcripts have been mechanically inoculated onto leaves of four weeks old Nicotiana benthamiana plants together with the TNV helper virus.

All infected plants were continuously scored for pds inactivation, which resulted in a phenotype showing leaf bleaching. For all inoculations necrotic lesions were observed for the inoculated leaves after 2 days post inoculation (p.i.). Within a week, necrotic lesions also occurred in upper leaves indicating the systemic spread of the viruses in N.benthamiana. In most infected plants, the virus symptoms have been severe but plants survived for many weeks.

Only plants, which have been infected with the hybrid vectors pIF9 and pIF12 carrying pds fragments, showed on top of the virus symptoms additional phenotypic changes. Approximately 4 weeks p.i., green upper leaves, which did not show any virus symptoms, developed bleached spots scattered all over the leaves. The bleaching was progressive and was not accompanied by necrotic lesions. Within another three weeks, the size of the spots increased constantly and the color changed from pale green to yellow to pale white-yellow. These symptoms have never been observed in plants, on which TNV/STNV wild type or TNV/STNV-deletion mutant control inoculations were carried out. Thus, this phenotype indicates the functional knockout of pds in N. benthamiana plants after infection with pIF9 and pIF12 hybrid viral vectors.

In RNA gel blot analyses performed with total RNA preparations of green and bleached spots of upper leaves of plants showing a FKO phenotype no chimeric STNV virus RNA and only very low levels of TNV helper virus RNA could be detected. This indicates that virus induced gene silencing of pds in a specific tissue does not need to be accompanied by high levels of virus RNA.

### Example 4: Inactivation of phytoene desaturase in Petunia hybrida using a TMV/STMV hybrid vector system.

A TMV/STMV hybrid vector system was used for the functional inactivation of a constitutively expressed endogenous plant gene. Therefore, the following STMV hybrid vectors have been constructed:
pVE293 carrying the following operationally linked elements:
   - a T7 RNA polymerase promoter comprising nucleotide 402 to 420 of Genbank Accession Number M77811
   - a nucleic acid comprising the nucleotide sequence from nucleotide 1 to 197 of STMV of SEQ ID No 4 or Genbank Accession Number M25782 (STMV leader)
   - a nucleic acid comprising the origin of assembly (OAS) of TMV-U2 from nucleotide 5430 to 5505 of SEQ ID No 5 or Genbank Accession Number M34077 ("short" TMV OAS)
   - a fragment of the tomato phytoene desaturase (pds) cDNA comprising nucleotide 1021 to 1671 of SEQ ID No 6 or Genbank Accession Number X59948
   - a nucleic acid comprising the nucleotide sequence from nucleotide 604 to 1058 of STMV of SEQ ID No 4 or Genbank Accesion Number M25782 (STMV trailer)
   - a SP6 RNA polymerase promoter comprising nucleotide 143 to 124 of Genbank Accession Number X65308
pVE294 carrying the following operationally linked elements:
   - a T7 RNA polymerase promoter comprising nucleotide 402 to 420 of Genbank Accession Number M77811
   - a nucleic acid comprising the nucleotide sequence from nucleotide 1 to 197 of STMV of SEQ ID No 4 or Genbank Accession Number M25782 (STMV leader)
   - a nucleic acid comprising the origin of assembly (OAS) of TMV-U2 from nucleotide 5441 to 5849 of SEQ ID No 2 or Genbank Accession Number M34077 ("long" TMV OAS)
   - a fragment of the tomato phytoene desaturase (pds) cDNA comprising nucleotide 1021 to 1671 of SEQ ID No 6 or Genbank Accession Number X59948
   - a nucleic acid comprising the nucleotide sequence from nucleotide 604 to 1058 of STMV of SEQ ID No 4 or Genbank Accesion Number M25782 (STMV trailer)
   - a SP6 RNA polymerase promoter comprising nucleotide 143 to 124 of Genbank Accession Number X65308

Infective chimeric transcripts have been synthesized in vitro using T7 RNA polymerase with the linearized plasmid DNAs of the described pVE293 and pVE294 hybrid vectors as templates using standard procedures. Control in vitro transcripts have been synthesized on linearized plasmid DNAs of the precursor plasmids of pVE293 and pVE294 without the pds fragment insert and on linearized plasmid DNA of the infective clone STMV-10 of the wild type.

The in vitro transcripts have been mechanically inoculated onto leaves of three months old Petunia hybrida V26 plants together with the TMV-U2 helper virus.

The infected plants have been continuously scored for pds inactivation, which resulted in a phenotype showing leaf bleaching. The infection of petunia plants with TMV/STMV caused the occurrence of crinkled leaves starting from the infected branches but quickly progressing systemically throughout the plant. The plants did survive the infections and often showed recovery phenotypes with almost no symptoms.

Only the plants, which have been infected with the hybrid vectors pVE293 and pVE294 carrying the pds fragment, showed in addition to the virus symptoms, additional phenotypic changes. In case of TMV/pVE293 infections, the infected branches developed young leaves with bleached sectors around the veins and at the tip of the leaves. This bleaching was progressive and independent from the presence of virus symptoms. Some of the leaves were fully bleached and progressively changed into almost white color. This phenotype was restricted to leaves of the infected branches. In case of TMV/pVE294 infections a similar bleaching of young leaves was observed for all developing branches but not for branches carrying already terminal flower buds. The bleaching started again around the veins and leaf tips but proceeded quickly producing variable leaf variegation patterns. This phenotype has never been observed in control inoculations with wild type viruses or deletion mutants. Therefore this phenotype indicates the functional knockout of pds in Petunia hybrida plants after infection with pVE293 and pVE294 viral hybrid vectors.

In RNA gel blot analyses performed with total RNA preparations of green and bleached spots of upper leaves of plants showing a FKO phenotype no chimeric STMV virus RNA and only low levels of TMV helper virus RNA could be detected. This indicates that virus induced gene silencing of pds in a specific tissue does not need to be accompanied by high levels of virus RNA.

### REFERENCES

Ausubel et al. (1994) Current Protocols in Molecular Biology, Current Protocols, USA.
Baulcombe (1996) Plant Cell 8: 1833-1844
Baulcombe et al. (1998) JIC &SL Annual Report 1996/1997
Bringloe et al. (1998) 79: 1539-1546
Chapman (1991) PhD dissertation, University of Cambridge, UK
Coutts et al. (1991) J. Gen. Virology 72: 1521-1529
Danthinne et al.(1991) Virology 185: 605-614
Depicker and Van Montagu (1997) Curr. Opin. Cell. Biol. 9: 373-382
English et al. (1996) Plant Cell 8, 179-188
Hamilton et al. (1998) The Plant Journal 15(6): 737-746
Kempin et al. (1997) Nature 389: 802-803
Kumagai et al. (1995) Proc. Natl. Acad. Sci USA 92: 1679-1683
Meulewaeter et al (1990) Virology 177:699-709
Pereira and Aerts (1998) Methods in Molecular Biology 82 Eds. Martinez-Zapatar and Salinas, Humana Press, NJ
R.D.D. Croy (1993) Plant Molecular Biology Labfax jointly published by BIOS Scientific Publications Ltd (UK) and Blackwell Scientific Publications, UK.
Prahasar et al. (1996) Proc. Natl. Acad. Sci USA 93: 659-663
Ruiz et al. (1998) The Plant Cell 10: 937-946
Sambrook et al. (1989) Molecular Cloning: A Laboratory Manual, Second Edition, Cold Spring Harbor Laboratory Press, NY
Stam et al. (1997) Ann. Botan. 79:3-12
Takayuki et al. (1995) The Plant Journal 8(5):771-776
Walkey (1985) Applied Virology, William Heinemann Ltd, London
Waterhouse et al. (1998) Proc. Natl. Acad. Sci USA 95: 13959-13964
Wellink et al. (1998) Abstract presented at the Joint Meeting of Arbeitskreis Virologie and Nederlandse Kring voor Plantenvirologie in Wageningen, The Netherlands. November 12 and 13, 1998.
Wilbur and Lipmann (1983) Proc. Nat. Acad. Sci. USA 80: 726
Ysebaert et al. (1980) J. Mol. Biol. 143: 273-287

## Claims

1. A method for the introduction of inhibitory RNA in the cytoplasm of plant cells, said inhibitory RNA reducing or abolishing the expression of a target gene in said plant cells, said method comprising:
a) introducing into said plant cells, a viral RNA vector comprising said inhibitory RNA or comprising a chimeric nucleic acid which when transcribed yields said inhibitory RNA, wherein said inhibitory RNA comprises a sense RNA or an antisense RNA comprising a nucleotide sequence of at least 100 nucleotides in length having at least 75% sequence identity to the nucleotide sequence of said target gene in said plant cell, and wherein said viral RNA vector comprises at least cis elements from a satellite RNA virus recognized by a replicase from a corresponding helper virus and is capable of replicating in a plant cell only when provided externally with essential functions required for said replication; and
b) introducing said corresponding helper virus into said plant cell.

2. The method of claim 1, wherein said inhibitory RNA comprises sense RNA of at least 100 nucleotides in length.

3. The method of claim 1, wherein said inhibitory RNA comprises antisense RNA of at least 100 nucleotides in length.

4. The method of claim 1, wherein said inhibitory RNA comprises a complementary stretch of at least 50 nucleotides of sense and antisense RNA.

5. The method of claim 1 or 4, wherein said inhibitory RNA comprises a complementary stretch of at least 100 nucleotides of sense and antisense RNA.

6. The method of any one of claims 1 to 5, wherein said viral RNA vector comprises said cis elements from STMV and wherein said helper virus is tobacco mosaic virus.

7. The method of any one of claims 1 to 5 , wherein said viral RNA vector comprises said cis elements from satellite tobacco necrosis virus and further comprises an origin of assembly of tobacco mosaic virus and wherein said helper virus is a tobacco necrosis virus comprising a coat protein gene of tobacco mosaic virus.

8. The method of claim 7, wherein said satellite RNA virus is satellite tobacco necrosis virus strain 1 or 2 and said TNV virus is TNV-A.

9. The method of claim 7, wherein said satellite RNA virus is STNV-C and said TNV virus is TNV-D.

10. The method of any one of claims 1 to 9, wherein said plant is selected from Nicotiana spp, Oryza sativa, Zea mays, Brassica spp., Gossypium spp., Triticum spp., Arabidopsis spp. or Petunia spp.

11. A kit for introduction of inhibitory RNA in the cytoplasm of a plant cell, said inhibitory RNA reducing or abolishing the expression of a target gene in said plant cell, said kit comprising
a) a viral RNA vector comprising said inhibitory RNA or a chimeric nucleic acid which when transcribed yields said inhibitory RNA, wherein said inhibitory RNA comprises a sense RNA or an antisense RNA comprising a nucleotide sequence of at least 100 nucleotides in length having at least 75% sequence identity to the nucleotide sequence of said target gene in said plant cell, and wherein said viral RNA vector comprises at least cis elements from a satellite RNA virus recognized by a replicase from a corresponding helper virus and is capable of replicating in a plant cell only when provided externally with essential functions required for said replication; and
b) said corresponding helper virus.

12. The kit of claim 11, wherein said inhibitory RNA comprises sense RNA of at least 100 nucleotides in length.

13. The kit of claim 11, wherein said inhibitory RNA comprises antisense RNA of at least 100 nucleotides in length.

14. The kit of claim 11, wherein said inhibitory RNA comprises a complementary stretch of at least 50 nucleotides of sense and antisense RNA.

15. The kit of claim 11 or 14, wherein said inhibitory RNA comprises a complementary stretch of at least 100 nucleotides of sense and antisense RNA.

16. The kit of any one of claims 11 to 15, wherein said viral RNA vector comprises said cis elements from STMV, and wherein said corresponding helper virus is tobacco mosaic virus.

17. The kit of any one of claim 11 to 15, wherein said viral RNA vector comprises said cis elements from satellite tobacco necrosis virus and further comprises an origin of assembly of tobacco mosaic virus and wherein said corresponding helper virus is a tobacco necrosis virus comprising a coat protein gene of tobacco mosaic virus.

18. The kit of claim 17, wherein said satellite RNA virus is satellite tobacco necrosis vector strain 1 or 2 and said corresponding TNV virus is TNV-A.

19. The kit of claim 17, wherein said satellite RNA virus is STNV-C and said TNV virus is TNV-D.

20. A method for isolating genes involved in the determination of a trait or phenotype of a plant species, said method comprising
a) identifying a set of nucleic acid sequences of genes, whose expression is correlated with a trait of interest,
b) creating a library of gene silencing constructs in a viral RNA vector, said viral RNA vector comprising at least cis elements from a satellite RNA virus recognized by a replicase from a corresponding helper virus and capable of replicating in a plant cell only when provided externally with essential functions required for said replication, and said gene silencing constructs being nucleic acids, which when transcribed yield RNA molecules comprising sense RNA or antisense RNA or both, said sense or antisense RNA comprising a nucleotide sequence of at least 100 nucleotides having at least 75% sequence identity with said nucleic acid sequences of said genes, whose expression is correlated with said trait of interest;
c) infecting a collection of individual plants of said plant species with said library of gene silencing constructs and with a corresponding helper virus or helper virus RNA, whereby each plant is infected with at least one member of said library and with said helper virus or helper virus RNA;
d) identifying a plant wherein said trait or phenotype is altered, using an assay adapted to said trait or phenotype;
e) isolating said gene involved in the determination of said trait or phenotype in said plant species, from said library based on the nucleotide sequence to which said gene silencing construct in said identified plant was targeted.

21. The method of claim 20, wherein said satellite RNA virus is satellite tobacco mosaic virus.

22. The method of claim 21, wherein said viral RNA vector comprises an origin of assembly of tobacco mosaic virus.

23. The method of any one of claims 20 to 22, wherein said helper virus is tobacco mosaic virus.

24. The method of claim 20, wherein said satellite RNA virus is satellite tobacco necrosis virus.

25. The method of claim 24, wherein said viral RNA vector comprises an origin of assembly of tobacco mosaic virus.

26. The method of any one of claims 20, 24 or 25, wherein said helper virus is tobacco necrosis virus comprising a coat protein gene of tobacco mosaic virus.

27. The method of claim 26, wherein said satellite RNA virus is satellite tobacco necrosis virus strain 1 or 2 and said TNV virus is TNV-A.

28. The method of claim 26, wherein said satellite RNA virus is STNV-C and said TNV virus is TNV-D.

29. The method of any one of claim 20 to 28, wherein said gene-silencing constructs comprise antisense RNA of at least 100 nucleotides in length.

30. The method of any one of claims 20 to 28, wherein said gene-silencing constructs comprise sense RNA of at least 100 nucleotides in length.

31. The method of any one of claims 20 to 28, wherein said gene-silencing constructs comprise a complementary stretch of at least 50 nucleotide of sense and antisense RNA.

32. The method of claim 31, wherein said gene-silencing constructs comprise a complementary stretch of at least 100 nucleotides of sense and antisense RNA.

33. The method of claim 31 or 32, wherein said gene-silencing constructs comprise at least two copies of part of the nucleotide sequences of said collection of nucleic acids, said copies being in inverted repeat.

34. A method for the isolation of a nucleic acid with a specific function from a collection of nucleic acids, said collection of nucleic acids being **characterized in that** variation in the expression pattern of said nucleic acids is correlated with variation in a trait/phenotype of a plant harboring said nucleic acids, said method comprising the steps of
a) creating a library of gene silencing constructs in a viral RNA vector, said viral RNA vector comprising at least cis elements from a satellite RNA virus recognized by a replicase from a corresponding helper virus and capable of replicating in a plant cell only when provided externally with essential functions required for said replication, and said gene silencing constructs being nucleic acids, which when transcribed yield RNA molecules comprising sense RNA or antisense RNA or both, said sense or antisense RNA comprising a nucleotide sequence of at least 100 nucleotides having at least 75% sequence identity with the nucleotide sequence of said nucleic acids, the variation in the expression pattern of which is correlated with variation in a trait/phenotype of a plant harboring said nucleic acids;
b) infecting a collection of plants with said library of gene silencing constructs and with said corresponding helper virus or helper virus RNA, whereby each plant is infected with at least one member of said library and with said helper virus or helper virus RNA;
c) identifying plants with altered trait or phenotype using an assay adapted to said trait or phenotype.

35. The method of claim 34, further comprising the step of isolating said nucleic acid with said specific function from said identified plant with altered trait or phenotype.

36. A method for determining the function encoded by a nucleic acid comprising a known nucleotide sequence in a plant, said method comprising
a) providing a viral RNA vector, said viral RNA vector comprising at least cis elements from a satellite RNA virus recognized by a replicase from a corresponding helper virus and capable of replicating in a plant cell only when provided externally with essential functions required for said replication, comprising a gene-silencing construct being a nucleic acid, which when transcribed yields RNA molecules comprising sense RNA or antisense RNA or both, said sense or antisense RNA comprising a nucleotide sequence of at least 100 nucleotides having at least 75% sequence identity with said known nucleotide sequence;
b) infecting said plant with said viral RNA vector and said corresponding helper virus;
c) identifying an altered trait or phenotype of said co-infected plant.

37. A method for identifying essential genes in a plant, comprising
a) creating a library of random gene-silencing constructs by cloning random DNA or cDNA fragments of said plant of at least 100 nucleotides in length in a cDNA copy of a viral RNA vector comprising at least cis elements from a satellite RNA virus recognized by a replicase from a corresponding helper virus and capable of replicating in a plant cell only when provided externally with essential functions required for said replication;
b) infecting a plant with at least one member of said library and with said corresponding helper virus;
c) identifying plants developing a gene-silencing-construct-associated phenotype.

38. The method of claim 37, further comprising the step of isolating the viral RNA vector from the tissue exhibiting said gene-silencing-construct-associated phenotype.

39. The method of claim 37, wherein said library is created by cloning random DNA fragments of said plant of at least 100 nucleotides in length in a cDNA copy of the viral RNA vector.

40. The method of claim 37, wherein said library is created by cloning random cDNA fragments of said plant of at least 100 nucleotides in length in a cDNA copy of the viral RNA vector.

41. The method of claim 37, wherein said library is created by cloning random duplicated cDNA fragments of said plant of at least 100 nucleotides in length in inverted repeat.

42. The method of any one of claims 37 to 40, wherein said satellite RNA virus is STMV, and wherein said helper virus is tobacco mosaic virus.

43. The method of any one of claims 37 to 40, wherein said satellite RNA virus is satellite tobacco necrosis virus and comprises an origin of assembly of tobacco mosaic virus and wherein said helper virus is tobacco necrosis virus comprising a coat protein gene of tobacco mosaic virus.

44. The method of claim 42, wherein said satellite RNA virus is satellite tobacco necrosis virus strain 1 or 2 and said TNV virus is TNV-A.

45. The method of claim 42, wherein said satellite RNA virus is STNV-C and said TNV virus is TNV-D.

## Patentansprüche

1. Verfahren zum Einführen von inhibitorischer RNA in das Zytoplasma von Pflanzenzellen, wobei die genannte inhibitorische RNA die Expression eines Zielgens in diesen genannten Pflanzenzellen verringert oder aufhebt, wobei das genannte Verfahren folgendes umfaßt:
a) Einführen eines Virus-RNA-Vektors, der diese genannte inhibitorische RNA umfaßt oder der eine chimäre Nukleinsäure, die, wenn sie transkribiert wird, die genannte inhibitorische RNA ergibt, umfaßt, in die genannten Pflanzenzellen, wobei die genannte inhibitorische RNA eine sense-RNA oder eine antisense-RNA umfassend eine Nukleotidsequenz mit einer Länge von mindestens 100 Nukleotiden und mindestens 75% Sequenzidentität zu der Nukleotidsequenz des genannten Zielgens in der genannten Pflanzenzelle umfaßt, und wobei der genannte Virus-RNA-Vektor mindestens cis-Elemente von einem Satelliten-RNA-Virus, das von einer Replikase von einem entsprechenden Helfervirus erkannt wird, umfaßt und nur dann zur Replikation in einer Pflanzenzelle befähigt ist, wenn ihm essentielle Funktionen, die für die genannte Replikation erforderlich sind, von außen bereitgestellt werden; und
b) Einführen des genannten entsprechenden Helfervirus in die genannte Pflanzenzelle.

2. Verfahren nach Anspruch 1, wobei die genannte inhibitorische RNA sense-RNA mit einer Länge von mindestens 100 Nukleotiden umfaßt.

3. Verfahren nach Anspruch 1, wobei die genannte inhibitorische RNA antisense-RNA mit einer Länge von mindestens 100 Nukleotiden umfaßt.

4. Verfahren nach Anspruch 1, wobei die genannte inhibitorische RNA einen komplementären Abschnitt mit mindestens 50 Nukleotiden sense- und antisense-RNA umfaßt.

5. Verfahren nach Anspruch 1 oder 4, wobei die genannte inhibitorische RNA einen komplementären Abschnitt mit mindestens 100 Nukleotiden sense- und antisense-RNA umfaßt.

6. Verfahren nach einem der Ansprüche 1 bis 5, wobei der genannte Virus-RNA-Vektor die genannten cis-Elemente aus dem STMV umfaßt und wobei es sich bei dem genannten Helfervirus um das Tabak-Mosaik-Virus handelt.

7. Verfahren nach einem der Ansprüche 1 bis 5, wobei der genannte Virus-RNA-Vektor die genannten cis-Elemente aus dem Tabak-Nekrose-Satelliten-Virus umfaßt und weiterhin einen Assemblierungsursprung aus dem Tabak-Mosaik-Virus umfaßt und wobei es sich bei dem genannten Helfervirus um ein Tabak-Nekrose-Virus umfassend ein Hüllproteingen des Tabak-Mosaik-Virus handelt.

8. Verfahren nach Anspruch 7, wobei es sich bei dem genannten Satelliten-RNA-Virus um den Tabak-Nekrose-Satelliten-Virus-Stamm 1 oder 2 und bei dem genannten TNV-Virus um TNV-A handelt.

9. Verfahren nach Anspruch 7, wobei es sich bei dem genannten Satelliten-RNA-Virus um STNV-C und bei dem genannten TNV-Virus um TNV-D handelt.

10. Verfahren nach einem der Ansprüche 1 bis 9, wobei die genannte Pflanze aus der Gruppe Nicotiana spp, Oryza sativa, Zea mays, Brassica spp., Gossypium spp., Triticum spp., Arabidopsis spp. oder Petunia spp. ausgewählt ist.

11. Kit zum Einführen von inhibitorischer RNA in das Zytoplasma einer Pflanzenzelle, wobei die genannte inhibitorische RNA die Expression eines Zielgens in der genannten Pflanzenzelle verringert oder aufhebt, wobei das genannte Kit folgendes umfaßt:
a) einen Virus-RNA-Vektor, der die genannte inhibitorische RNA umfaßt oder der eine chimäre Nukleinsäure, die, wenn sie transkribiert wird, die genannte inhibitorische RNA ergibt, umfaßt, wobei die genannte inhibitorische RNA eine sense-RNA oder eine antisense-RNA umfassend eine Nukleotidsequenz mit einer Länge von mindestens 100 Nukleotiden und mindestens 75% Sequenzidentität zu der Nukleotidsequenz des genannten Zielgens in der genannten Pflanzenzelle umfaßt, und wobei der genannte Virus-RNA-Vektor mindestens cis-Elemente von einem Satelliten-RNA-Virus, das von einer Replikase von einem entsprechenden Helfervirus erkannt wird, umfaßt und nur dann zur Replikation in einer Pflanzenzelle befähigt ist, wenn ihm essentielle Funktionen, die für die genannte Replikation erforderlich sind, von außen bereitgestellt werden; und
b) das genannte entsprechende Helfervirus.

12. Kit nach Anspruch 11, wobei die genannte inhibitorische RNA sense-RNA mit einer Länge von mindestens 100 Nukleotiden umfaßt.

13. Kit nach Anspruch 11, wobei die genannte inhibitorische RNA antisense-RNA mit einer Länge von mindestens 100 Nukleotiden umfaßt.

14. Kit nach Anspruch 11, wobei die genannte inhibitorische RNA einen komplementären Abschnitt mit mindestens 50 Nukleotiden sense- und antisense-RNA umfaßt.

15. Kit nach Anspruch 11 oder 14, wobei die genannte inhibitorische RNA einen komplementären Abschnitt mit mindestens 100 Nukleotiden sense- und antisense-RNA umfaßt.

16. Kit nach einem der Ansprüche 11 bis 15, wobei der genannte Virus-RNA-Vektor die genannten cis-Elemente aus dem STMV umfaßt und wobei es sich bei dem genannten Helfervirus um das Tabak-Mosaik-Virus handelt.

17. Kit nach einem der Ansprüche 11 bis 15, wobei der genannte Virus-RNA-Vektor die genannten cis-Elemente aus dem Tabak-Nekrose-Satelliten-Virus umfaßt und weiterhin einen Assemblierungsursprung aus dem Tabak-Mosaik-Virus umfaßt und wobei es sich bei dem genannten Helfervirus um ein Tabak-Nekrose-Virus umfassend ein Hüllproteingen des Tabak-Mosaik-Virus handelt.

18. Kit nach Anspruch 17, wobei es sich bei dem genannten Satelliten-RNA-Virus um den Tabak-Nekrose-Satelliten-Virus-Stamm 1 oder 2 und bei dem genannten TNV-Virus und TNV-A handelt.

19. Kit nach Anspruch 17, wobei es sich bei dem genannten Satelliten-RNA-Virus um STNV-C und bei dem genannten TNV-Virus um TNV-D handelt.

20. Verfahren zum Isolieren von Genen, die an der Bestimmung eines Merkmals oder Phänotyps einer Pflanzenart beteiligt sind, wobei das genannte Verfahren folgendes umfaßt:
a) Identifizieren eines Satzes von Nukleinsäuresequenzen von Genen, deren Expression mit einem interessierenden Merkmal korreliert ist,
b) Erzeugen einer Bibliothek von Geninaktivierungskonstrukten in einem Virus-RNA-Vektor, wobei der genannte Virus-RNA-Vektor mindestens cis-Elemente von einem Satelliten-RNA-Virus, das von einer Replikase von einem entsprechenden Helfervirus erkannt wird, umfaßt und nur dann zur Replikation in einer Pflanzenzelle befähigt ist, wenn ihm essentielle Funktionen, die für die genannte Replikation erforderlich sind, von außen bereitgestellt werden, und wobei es sich bei den genannten Geninaktivierungskonstrukten um Nukleinsäuren handelt, die, wenn sie transkribiert werden, RNA-Moleküle umfassend sense-RNA oder antisense-RNA oder beide ergeben, wobei die genannte sense- oder antisense-RNA eine Nukleotidsequenz von mindestens 100 Nukleotiden mit mindestens 75% Sequenzidentität mit genannten Nukleinsäuresequenzen von genannten Genen, deren Expression mit dem genannten interessierenden Merkmal korreliert ist, umfaßt;
c) Infizieren einer Gruppe von Einzelpflanzen dieser genannten Pflanzenart mit der genannten Bibliothek von Geninaktivierungskonstrukten und mit einem entsprechenden Helfervirus oder einer entsprechenden Helfervirus-RNA, wobei jede Pflanze mit mindestens einem Mitglied der genannten Bibliothek und mit dem genannten Helfervirus bzw. der genannten Helfervirus-RNA infiziert wird;
d) Identifizieren einer Pflanze, in der das genannte Merkmal bzw. der genannte Phänotyp verändert ist, unter Verwendung eines Tests, der an das genannte Merkmal bzw. den genannten Phänotyp angepaßt ist;
e) Isolieren des genannten Gens, das an der Bestimmung des genannten Merkmals oder Phänotyps in der genannten Pflanzenart beteiligt ist, aus der genannten Bibliothek, die auf der Nukleotidsequenz beruht, die dem genannten Geninaktivierungskonstrukt in der genannten identifizierten Pflanze als Ziel gedient hat.

21. Verfahren nach Anspruch 20, wobei es sich bei dem genannten Satelliten-RNA-Virus um das Satelliten-Tabak-Mosaik-Virus handelt.

22. Verfahren nach Anspruch 21, wobei der genannte Virus-RNA-Vektor einen Assemblierungsursprung des Tabak-Mosaik-Virus umfaßt.

23. Verfahren nach einem der Ansprüche 20 bis 22, wobei es sich bei dem genannten Helfervirus um das Tabak-Mosaik-Virus handelt.

24. Verfahren nach Anspruch 20, wobei es sich bei dem genannten Satelliten-RNA-Virus um das Tabak-Nekrose-Satelliten-Virus handelt.

25. Verfahren nach Anspruch 24, wobei der genannte Virus-RNA-Vektor einen Assemblierungsursprung des Tabak-Mosaik-Virus umfaßt.

26. Verfahren nach einem der Ansprüche 20, 24 oder 25, wobei es sich bei dem genannten Helfervirus um das Tabak-Nekrose-Virus umfassend ein Hüllproteingen des Tabak-Mosaik-Virus handelt.

27. Verfahren nach Anspruch 26, wobei es sich bei dem genannten Satelliten-RNA-Virus um den Tabak-Nekrose-Satelliten-Virus-Stamm 1 oder 2 und bei dem genannten TNV-Virus und TNV-A handelt.

28. Verfahren nach Anspruch 26, wobei es sich bei dem genannten Satelliten-RNA-Virus um STNV-C und bei dem genannten TNV-Virus um TNV-D handelt.

29. Verfahren nach einem der Ansprüche 20 bis 28, wobei die genannten Geninaktivierungskonstrukte antisense-RNA mit einer Länge von mindestens 100 Nukleotiden umfassen.

30. Verfahren nach einem der Ansprüche 20 bis 28, wobei die genannten Geninaktiverungskonstrukte sense-RNA mit einer Länge von mindestens 100 Nukleotiden umfassen.

31. Verfahren nach einem der Ansprüche 20 bis 28, wobei die genannten Geninaktiverungskonstrukte einen komplementären Abschnitt mit mindestens 50 Nukleotiden sense- und antisense-RNA umfassen.

32. Verfahren nach Anspruch 31, wobei die genannten Geninaktivierungskonstrukte einen komplementären Abschnitt von mindestens 100 Nukleotiden sense- und antisense-RNA umfassen.

33. Verfahren nach Anspruch 31 oder 32, wobei die genannten Geninaktivierungskonstrukte mindestens zwei Kopien eines Teils der Nukleotidsequenzen der genannten Gruppe von Nukleinsäuren umfassen, wobei die genannten Kopien in invertierten Sequenzwiederholungen vorliegen.

34. Verfahren zum Isolieren einer Nukleinsäure mit einer spezifischen Funktion aus einer Gruppe von Nukleinsäuren, wobei die genannte Gruppe von Nukleinsäuren **dadurch gekennzeichnet ist, daß** die Variation im Expressionsmuster der genannten Nukleinsäuren mit einer Variation in einem Merkmal/Phänotyp an der Pflanze, die die genannten Nukleinsäuren birgt, korreliert, wobei das genannte Verfahren die folgenden Schritte umfaßt:
a) Erzeugen einer Bibliothek von Geninaktivierungskonstrukten in einem Virus-RNA-Vektor, wobei der genannte Virus-RNA-Vektor mindestens cis-Elemente von einem Satelliten-RNA-Virus, das von einer Replikase von einem entsprechenden Helfervirus erkannt wird, umfaßt und nur dann zur Replikation in einer Pflanzenzelle befähigt ist, wenn ihm essentielle Funktionen, die für die genannte Replikation erforderlich sind, von außen bereitgestellt werden, und wobei es sich bei den genannten Geninaktivierungskonstrukten um Nukleinsäuren handelt, die, wenn sie transkribiert werden, RNA-Moleküle umfassend sense-RNA oder antisense-RNA oder beide ergeben, wobei die genannte sense- oder antisense-RNA eine Nukleotidsequenz von mindestens 100 Nukleotiden mit mindestens 75% Sequenzidentität mit den Nukleinsäuresequenzen von den genannten Genen, deren Variation im Expressionsmuster mit einer Variation in einem Merkmal/Phänotyp einer Pflanze, die die genannten Nukleinsäuren birgt, korreliert ist, umfaßt;
b) Infizieren einer Gruppe von Einzelpflanzen mit der genannten Bibliothek von Geninaktivierungskonstrukten und mit dem genannten entsprechenden Helfervirus oder einer entsprechenden Helfervirus-RNA, wobei jede Pflanze mit mindestens einem Mitglied der genannten Bibliothek und mit dem genannten Helfervirus bzw. der genannten Helfervirus-RNA infiziert wird;
c) Identifizieren von Pflanzen, in denen das genannte Merkmal bzw. der genannte Phänotyp verändert ist, unter Verwendung eines Tests, der an das genannte Merkmal bzw. den genannten Phänotyp angepaßt ist.

35. Verfahren nach Anspruch 34, das weiterhin den Schritt umfaßt, daß man die genannte Nukleinsäure mit der genannten spezifischen Funktion aus der genannten identifizierten Pflanze mit dem veränderten Merkmal oder Phänotyp isoliert.

36. Verfahren zur Bestimmung der Funktion, die von einer Nukleinsäure umfassend eine bekannte Nukleotidsequenz in einer Pflanze codiert wird, wobei das genannte Verfahren folgendes umfaßt:
a) Bereitstellen eines Virus-RNA-Vektors, wobei der genannte Virus-RNA-Vektor mindestens cis-Elemente von einem Satelliten-RNA-Virus, das von einer Replikase von einem entsprechenden Helfervirus erkannt wird, umfaßt und nur dann zur Replikation in einer Pflanzenzelle befähigt ist, wenn ihm essentielle Funktionen, die für die genannte Replikation erforderlich sind, von außen bereitgestellt werden, umfassend ein Geninaktivierungskonstrukt, bei dem es sich um eine Nukleinsäure handelt, die, wenn sie transkribiert wird, RNA-Moleküle umfassend sense-RNA oder antisense-RNA oder beide ergibt, wobei die genannte sense- oder antisense-RNA eine Nukleotidsequenz von mindestens 100 Nukleotiden mit mindestens 75% Sequenzidentität mit der benannten Nukleotidsequenz umfaßt;
b) Infizieren der genannten Pflanze mit dem genannten Virus-RNA-Vektor und dem genannten entsprechenden Helfervirus;
c) Identifizieren eines veränderten Merkmals oder Phänotyps der genannten coinfizierten Pflanze.

37. Verfahren zum Identifizieren von essentiellen Genen in einer Pflanze, umfassend die folgenden Schritte:
a) Erzeugung einer Bibliothek von zufallsmäßigen Geninaktivierungskonstrukten durch Klonieren von zufallsmäßigen DNA- oder cDNA-Fragmenten der genannten Pflanze mit einer Länge von mindestens 100 Nukleotiden in eine cDNA-Kopie eines Virus-RNA-Vektors umfassend mindestens cis-Elemente von einem Satelliten-RNA-Virus, das von einer Replikase von einem entsprechenden Helfervirus erkannt wird und nur dann zur Replikation in einer Pflanzenzelle befähigt ist, wenn ihm essentielle Funktionen, die für die genannte Replikation erforderlich sind, von außen bereitgestellt werden;
b) Infizieren einer Pflanze mit mindestens einem Mitglied der genannten Bibliothek und mit dem genannten entsprechenden Helfervirus;
c) Identifizieren von Pflanzen, die einen Phänotyp entwickeln, der mit einem Geninaktivierungskonstrukt assoziiert ist.

38. Verfahren nach Anspruch 37, das weiterhin den Schritt des Isolierens des Virus-RNA-Vektors aus dem Gewebe, das den genannten Phänotyp, der mit dem Geninaktivierungskonstrukt assoziiert ist, aufweist, umfaßt.

39. Verfahren nach Anspruch 37, wobei die genannte Bibliothek durch Klonieren von zufallsmäßigen DNA-Fragmenten der genannten Pflanze mit einer Länge von mindestens 100 Nukleotiden in eine cDNA-Kopie des Virus-RNA-Vektors erzeugt wird.

40. Verfahren nach Anspruch 37, wobei die genannte Bibliothek durch Klonieren von zufallsmäßigen cDNA-Fragmenten der genannten Pflanze mit einer Länge von mindestens 100 Nukleotiden in eine cDNA-Kopie des Virus-RNA-Vektors erzeugt wird.

41. Verfahren nach Anspruch 37, wobei die genannte Bibliothek dadurch erzeugt wird, daß man zufallsmäßig verdoppelte cDNA-Fragmente der genannten Pflanze mit einer Länge von mindestens 100 Nukleotiden als invertierte Sequenzwiederholungen kloniert.

42. Verfahren nach einem der Ansprüche 37 bis 40, wobei es sich bei dem genannten Satelliten-RNA-Virus um STMV und bei dem genannten Helfervirus um das Tabak-Mosaik-Virus handelt.

43. Verfahren nach einem der Ansprüche 37 bis 40, wobei das genannte Satelliten-RNA-Virus das Tabak-Nekrose-Satelliten-Virus ist und einen Assemblierungsursprung des Tabak-Mosaik-Virus umfaßt und wobei das genannte Helfervirus das Tabak-Nekrose-Virus umfassend ein Hüllproteingen des Tabak-Mosaik-Virus ist.

44. Verfahren nach Anspruch 42, wobei es sich bei dem genannten Satelliten-RNA-Virus um den Tabak-Nekrose-Satelliten-Virus-Stamm 1 oder 2 und bei dem genannten TNV-Virus um TNV-A handelt.

45. Verfahren nach Anspruch 42, wobei es sich bei dem genannten Satelliten-RNA-Virus um STNV-C und bei dem genannten TNV-Virus um TNV-D handelt.

## Revendications

1. Procédé destiné à introduire un ARN inhibiteur dans le cytoplasme de cellules végétales, ledit ARN inhibiteur réduisant ou supprimant l'expression d'un gène cible dans lesdites cellules végétales, ledit procédé comprenant les étapes consistant à :
a) introduire dans lesdites cellules végétales un vecteur à ARN viral, comprenant ledit ARN inhibiteur ou comprenant un acide nucléique chimérique qui, lorsqu'il est transcrit, produit ledit ARN inhibiteur, dans lequel ledit ARN inhibiteur comprend un ARN sens ou un ARN antisens, qui comprend une séquence nucléotidique d'une longueur de 100 nucléotides au moins ayant une identité de séquence d'au moins 75% avec la séquence nucléotidique dudit gène cible dans ladite cellule végétale, et dans lequel ledit vecteur à ARN viral comprend au moins des éléments cis provenant d'un virus à ARN satellite reconnu par une réplicase, qui provient d'un virus auxiliaire correspondant, et qui est capable de se répliquer dans une cellule végétale seulement quand il est fourni de manière externe, avec des fonctions essentielles requises pour ladite réplication ; et
b) introduire ledit virus auxiliaire correspondant dans ladite cellule végétale.

2. Procédé selon la revendication 1, dans lequel ledit ARN inhibiteur comprend un ARN sens d'une longueur de 100 nucléotides au moins.

3. Procédé selon la revendication 1, dans lequel ledit ARN inhibiteur comprend un ARN antisens d'une longueur de 100 nucléotides au moins.

4. Procédé selon la revendication 1, dans lequel ledit ARN inhibiteur comprend un allongement complémentaire de 50 nucléotides au moins d'ARN sens et antisens.

5. Procédé selon la revendication 1 ou la 4, dans lequel ledit ARN inhibiteur comprend un allongement complémentaire de 100 nucléotides au moins d'ARN sens et antisens.

6. Procédé selon l'une quelconque des revendications 1 à 5, dans lequel ledit vecteur à ARN viral comprend lesdits éléments cis provenant d'un VSMT et dans lequel ledit virus auxiliaire est le virus de la mosaïque du tabac.

7. Procédé selon l'une quelconque des revendications 1 à 5, dans lequel ledit vecteur à ARN viral comprend lesdits éléments cis provenant d'un virus satellite de la nécrose du tabac et comprend en outre une origine d'assemblage du virus de la mosaïque du tabac et dans lequel ledit virus auxiliaire est un virus de la nécrose du tabac comprenant un gène de la protéine de surface du virus de la mosaïque du tabac.

8. Procédé selon la revendication 7, dans lequel ledit virus à ARN satellite est la souche 1 ou la 2 du virus satellite de la nécrose du tabac et ledit virus VNT est le VNT-A.

9. Procédé selon la revendication 7, dans lequel ledit virus à ARN satellite est le VSNT-C et ledit virus VNT est le VNT-D.

10. Procédé selon l'une quelconque des revendications 1 à 9, dans lequel ladite plante est choisie à partir de Nicotiana spp., Oryza sativa, Zea mays, Brassica spp., Gossypium spp., Triticum spp., Arabidopsis spp. ou Petunia spp.

11. Trousse destinée à introduire un ARN inhibiteur dans le cytoplasme d'une cellule végétale, ledit ARN inhibiteur réduisant ou supprimant l'expression d'un gène cible dans ladite cellule végétale, ladite trousse comprenant :
a) un vecteur à ARN viral, comprenant ledit ARN inhibiteur ou un acide nucléique chimérique qui, lorsqu'il est transcrit, produit ledit ARN inhibiteur, dans lequel ledit ARN inhibiteur comprend un ARN sens ou un ARN antisens, qui comprend une séquence nucléotidique d'une longueur de 100 nucléotides au moins ayant une identité de séquence d'au moins 75% avec la séquence nucléotidique dudit gène cible dans ladite cellule végétale, et dans lequel ledit vecteur à ARN viral comprend au moins des éléments cis provenant d'un virus à ARN satellite reconnu par une réplicase, qui provient d'un virus auxiliaire correspondant, et qui est capable de se répliquer dans une cellule végétale seulement quand il est fourni de manière externe, avec des fonctions essentielles requises pour ladite réplication ; et
b) ledit virus auxiliaire correspondant.

12. Trousse selon la revendication 11, dans laquelle ledit ARN inhibiteur comprend un ARN sens d'une longueur de 100 nucléotides au moins.

13. Trousse selon la revendication 11, dans lequel ledit ARN inhibiteur comprend un ARN antisens d'une longueur de 100 nucléotides au moins.

14. Trousse selon la revendication 11, dans lequel ledit ARN inhibiteur comprend un allongement complémentaire de 50 nucléotides au moins d'ARN sens et antisens.

15. Trousse selon la revendication 11 ou la 14, dans lequel ledit ARN inhibiteur comprend un allongement complémentaire de 100 nucléotides au moins d'ARN sens et antisens.

16. Trousse selon l'une quelconque des revendications 11 à 15, dans lequel ledit vecteur à ARN viral comprend lesdits éléments cis provenant d'un VSMT et dans lequel ledit virus auxiliaire correspondant est le virus de la mosaïque du tabac.

17. Trousse selon l'une quelconque des revendications 11 à 15, dans lequel ledit vecteur à ARN viral comprend lesdits éléments cis provenant d'un virus satellite de la nécrose du tabac et comprend en outre une origine d'assemblage du virus de la mosaïque du tabac et dans lequel ledit virus auxiliaire correspondant est un virus de la nécrose du tabac comprenant un gène de la protéine de surface du virus de la mosaïque du tabac.

18. Trousse selon la revendication 17, dans lequel ledit virus à ARN satellite est la souche 1 ou la 2 du vecteur satellite de la nécrose du tabac et ledit virus VNT est le VNT-A.

19. Trousse selon la revendication 17, dans lequel ledit virus à ARN satellite est le VSNT-C et ledit virus VNT est le VNT-D.

20. Procédé destiné à isoler des gènes impliqués dans la détermination d'un trait ou d'un phénotype d'espèce végétale, ledit procédé comprenant les étapes consistant à :
a) identifier un ensemble de séquences d'acide nucléique de gènes dont l'expression est en corrélation avec un trait d'intérêt,
b) créer une bibliothèque de construits à inactivation de gènes dans un vecteur à ARN viral, ledit vecteur à ARN viral comprenant au moins des éléments cis provenant d'un virus à ARN satellite reconnu par une réplicase, qui provient d'un virus auxiliaire correspondant, et qui est capable de se répliquer dans une cellule végétale seulement quand il est fourni de manière externe, avec des fonctions essentielles requises pour ladite réplication ; et lesdits construits à inactivation de gènes étant des acides nucléiques qui, lorsqu'ils sont transcrits, produisent des molécules d'ARN comprenant un ARN sens ou un ARN antisens ou bien les deux, ledit ARN sens ou antisens comprenant une séquence nucléotidique d'une longueur de 100 nucléotides au moins ayant une identité de séquence d'au moins 75% avec lesdites séquences d'acide nucléique desdits gènes, dont l'expression est en corrélation avec ledit trait d'intérêt ;
c) infecter une collection de plantes individuelles de ladite espèce végétale avec ladite bibliothèque de construits à inactivation de gènes et avec un virus auxiliaire correspondant ou un ARN de virus auxiliaire, moyennant quoi chaque plante est infectée avec au moins un membre de ladite bibliothèque et avec ledit virus auxiliaire ou ledit ARN de virus auxiliaire ;
d) identifier une plante, dans laquelle ledit trait ou ledit phénotype est altéré, en utilisant un dosage adapté au dit trait ou au dit phénotype ;
e) isoler ledit gène impliqué dans la détermination dudit trait ou dudit phénotype dans ladite espèce végétale à partir de ladite bibliothèque, sur la base de la séquence nucléotidique pour laquelle a été ciblé ledit construit à inactivation de gènes dans ladite plante identifiée.

21. Procédé selon la revendication 20, dans lequel ledit virus à ARN satellite est le virus satellite de la mosaïque du tabac.

22. Procédé selon la revendication 21, dans lequel ledit vecteur à ARN viral comprend une origine d'assemblage du virus de la mosaïque du tabac.

23. Procédé selon l'une quelconque des revendications 20 à 22, dans lequel ledit virus auxiliaire est le virus de la mosaïque du tabac.

24. Procédé selon la revendication 20, dans lequel ledit virus à ARN satellite est le virus satellite de la nécrose du tabac.

25. Procédé selon la revendication 24, dans lequel ledit vecteur à ARN viral comprend une origine d'assemblage du virus de la mosaïque du tabac.

26. Procédé selon l'une quelconque des revendications 20, 24 ou 25, dans lequel ledit virus auxiliaire est le virus de la nécrose du tabac comprenant un gène de la protéine de surface du virus de la mosaïque du tabac.

27. Procédé selon la revendication 26, dans lequel ledit virus à ARN satellite est la souche 1 ou la 2 du virus satellite de la nécrose du tabac et ledit virus VNT est le VNT-A.

28. Procédé selon la revendication 26, dans lequel ledit virus à ARN satellite est le VSNT-C et ledit virus VNT est le VNT-D.

29. Procédé selon l'une quelconque des revendications 20 à 28, dans laquelle lesdits construits à inactivation de gènes comprennent un ARN antisens d'une longueur de 100 nucléotides au moins.

30. Procédé selon l'une quelconque des revendications 20 à 28, dans laquelle lesdits construits à inactivation de gènes comprennent un ARN sens d'une longueur de 100 nucléotides au moins.

31. Procédé selon l'une quelconque des revendications 20 à 28, dans laquelle lesdits construits à inactivation de gènes comprennent un allongement complémentaire de 50 nucléotides au moins d'ARN sens et antisens.

32. Procédé selon la revendication 31, dans laquelle lesdits construits à inactivation de gènes comprennent un allongement complémentaire de 100 nucléotides au moins d'ARN sens et antisens.

33. Procédé selon la revendication 31 ou la 32, dans lequel lesdits construits à inactivation de gènes comprennent au moins deux copies d'une partie des séquences nucléotidiques de ladite collection d'acides nucléiques, lesdites copies étant dans une répétition inversée.

34. Procédé destiné à isoler un acide nucléique avec une fonction spécifique provenant d'une collection d'acides nucléiques, ladite collection d'acides nucléiques étant **caractérisée en ce qu'**une variation dans le schéma d'expression desdits acides nucléiques est en corrélation avec une variation dans le trait/phénotype d'une plante renfermant lesdits acides nucléiques, ledit procédé comprenant les étapes de :
a) création d'une bibliothèque de construits à inactivation de gènes dans un vecteur à ARN viral, ledit vecteur à ARN viral comprenant au moins des éléments cis provenant d'un virus à ARN satellite reconnu par une réplicase, qui provient d'un virus auxiliaire correspondant, et qui est capable de se répliquer dans une cellule végétale seulement quand il est fourni de manière externe, avec des fonctions essentielles requises pour ladite réplication ; et lesdits construits à inactivation de gènes étant des acides nucléiques qui, lorsqu'ils sont transcrits, produisent des molécules d'ARN comprenant un ARN sens ou un ARN antisens ou bien les deux, ledit ARN sens ou antisens comprenant une séquence nucléotidique d'une longueur de 100 nucléotides au moins ayant une identité de séquence d'au moins 75% avec la séquence nucléotidique desdits acides nucléiques, dont la variation dans le schéma d'expression est en corrélation avec la variation dans le trait/phénotype d'une plante renfermant lesdits acides nucléiques ;
b) infection d'une collection de plantes avec ladite bibliothèque de construits à inactivation de gènes et avec ledit virus auxiliaire correspondant ou un ARN de virus auxiliaire, moyennant quoi chaque plante est infectée avec au moins un membre de ladite bibliothèque et avec ledit virus auxiliaire ou ledit ARN de virus auxiliaire ;
c) identification de plantes avec un trait ou un phénotype altéré, en utilisant un dosage adapté au dit trait ou au dit phénotype.

35. Procédé selon la revendication 34, comprenant en outre l'étape d'isolement dudit acide nucléique avec ladite fonction spécifique provenant de ladite plante identifiée ayant un trait ou un phénotype altéré.

36. Procédé de détermination de la fonction codée par un acide nucléique comprenant une séquence nucléotidique connue dans une plante, ledit procédé comprenant les étapes consistant à :
a) fournir un vecteur à ARN viral, ledit vecteur à ARN viral comprenant au moins des éléments cis provenant d'un virus à ARN satellite reconnu par une réplicase, qui provient d'un virus auxiliaire correspondant, et qui est capable de se répliquer dans une cellule végétale seulement quand il est fourni de manière externe, avec des fonctions essentielles requises pour ladite réplication, comprenant un construit à inactivation de gènes étant un acide nucléique qui, lorsqu'il est transcrit, produit des molécules d'ARN comprenant un ARN sens ou un ARN antisens ou bien les deux, ledit ARN sens ou antisens comprenant une séquence nucléotidique d'une longueur de 100 nucléotides au moins ayant une identité de séquence d'au moins 75% avec ladite séquence nucléotidique connue ;
b) infecter ladite plante avec ledit vecteur à ARN viral et ledit virus auxiliaire correspondant ;
c) identifier un trait ou un phénotype altéré de ladite plante co-infectée.

37. Procédé d'identification de gènes essentiels dans une plante, comprenant les étapes consistant à :
a) créer une bibliothèque de construits aléatoires à inactivation de gènes par clonage de fragments d'ADN ou d'ADNc aléatoires de ladite plante, ayant une longueur de 100 nucléotides au moins, dans une copie d'ADNc d'un vecteur à ARN viral comprenant au moins des éléments cis provenant d'un virus à ARN satellite reconnu par une réplicase, qui provient d'un virus auxiliaire correspondant, et qui est capable de se répliquer dans une cellule végétale seulement quand il est fourni de manière externe, avec des fonctions essentielles requises pour ladite réplication ;
b) infecter une plante avec au moins un membre de ladite bibliothèque et avec ledit virus auxiliaire correspondant ;
c) identifier des plantes développant un phénotype associé au construit à inactivation de gènes.

38. Procédé, selon la revendication 37, comprenant en outre l'étape d'isolement du vecteur à ARN viral provenant du tissu qui présente ledit phénotype associé à un construit à inactivation de gènes.

39. Procédé selon la revendication 37, dans lequel ladite bibliothèque est créée en clonant des fragments aléatoires d'ADN de ladite plante, ayant une longueur de 100 nucléotides au moins, dans une copie d'ADNc du vecteur à ARN viral.

40. Procédé selon la revendication 37, dans lequel ladite bibliothèque est créée en clonant des fragments aléatoires d'ADNc de ladite plante, ayant une longueur de 100 nucléotides au moins, dans une copie d'ADNc du vecteur à ARN viral.

41. Procédé selon la revendication 37, dans lequel ladite bibliothèque est créée en clonant des fragments dupliqués d'ADNc de ladite plante, ayant une longueur de 100 nucléotides au moins, dans une répétition inversée.

42. Procédé selon l'une quelconque des revendications 37 à 40, dans lequel ledit virus à ARN satellite est un VSMT et dans lequel ledit virus auxiliaire est le virus de la mosaïque du tabac.

43. Procédé selon l'une quelconque des revendications 37 à 40, dans lequel ledit virus à ARN satellite est le virus satellite de la nécrose du tabac et comprend une origine d'assemblage du virus de la mosaïque du tabac et dans lequel ledit virus auxiliaire est un virus de la nécrose du tabac comprenant un gène de la protéine de surface du virus de la mosaïque du tabac.

44. Procédé selon la revendication 42, dans lequel ledit virus à ARN satellite est la souche 1 ou la 2 du virus satellite de la nécrose du tabac et ledit virus VNT est le VNT-A.

45. Procédé selon la revendication 42, dans lequel ledit virus à ARN satellite est le VSNT-C et ledit virus VNT est le VNT-D.
